# EUROPEAN PATENT APPLICATION

(11) **EP 1 992 631 A1**
(43) Date of publication of application: **19.11.2008**
(21) Application number: 07737952.7
(22) Date of filing: 07.03.2007
(51) Int. Cl.: C07F 15/00, C09K 11/06, H01L 51/50

(54) **METAL COMPLEX COMPOUND AND ORGANIC ELECTROLUMINESCENT DEVICE USING SAME**

(30) Priority: 09.03.2006 JP 2006063777; 06.09.2006 US 515777
(71) Applicant: Chuo University, Tokyo 192-0393 (JP); Idemitsu Kosan Co., Ltd., Chiyoda-ku Tokyo 100-8321 (JP)
(72) Inventor: HAGA, Masa-aki, Tokyo 112-0003 (JP); YANG, Li-fen, Tokyo 112-0003 (JP); OKUDA, Fumio, Sodegaura-shi Chiba 299-0293 (JP)
(74) Representative: Gille Hrabal Struck Neidlein Prop Roos
(86) International application number: PCT/JP2007/054438
(87) International publication number: WO 2007/102543

(57) **Abstract**

A metal-complex compound which comprises a tridentate chelate ligand having a specified partial structure. An organic electroluminescence device which comprises one or more organic thin film layers having at least one light emitting layer sandwiched between a pair of electrodes, wherein the organic thin film layer comprises the metal-complex compound, which emits light by applying an electric voltage between the pair of electrodes. The present invention provides an organic EL device which emits blue light of high purity and of short wavelength together with a metal-complex compound realizing the organic EL device.

## Description

### TECHNICAL FIELD

The present invention relates to a novel metal-complex compound and an organic electroluminescence device using the compound. Particularly, the present invention relates to an organic electroluminescence device ("electroluminescence" will be referred to as "EL", hereinafter) which emits blue light of high purity and of short wavelength with an enhanced efficiency of light emission, and to a metal-complex compound realizing it.

### BACKGROUND ART

The organic EL devices have been expected to be applied to color wide screen image display devices replacing liquid crystal display devices, and have been intensively developed. Recently, although displays using the organic EL devices have now been used in practical applications, full-color image display devices using the same are still in the course of development because they lack in sufficient light emitting property. In order for improving the light emitting property, very high-efficiency green organic light-emitting devices based on electrophosphorescence employing ortho metallized iridium complex (fac-tris(2-phenylpyridine)iridium) as a phosphorus light emitting material for the organic EL device are proposed. (refer to Non-patent literatures 1 and 2 which will be described later)
Because the current organic EL devices employing the electrophosphorescence are limited to emitting only green light, coverage as the color display devices is narrow. Therefore, it has been demanded to develop organic EL devices which emit light of different colors from green with improved light emission property. Regarding particularly with EL devices which emit blue light, those having an external quantum yield exceeding 5 % is not reported yet. Accordingly, an improvement in the EL devices which emit blue light, if possible, enables the display devices to display full colors or white light resultantly advancing toward practical use of phosphorus light EL device greatly.

Currently, developments about a compound having an iridium atom as an electrophosphorescent complex are actively carried out, and Compound A below is known as a material employable for an EL device which emits green light. On the other hand, Compound B below is known as a material for an EL device which emits blue light, however, the EL device employing Compound B is not practical in view points of both lifetime and efficiency of the device- Accordingly, it is necessary to develop another complex for EL devices which emit blue light, however, any material except Compound B has not been found yet now. 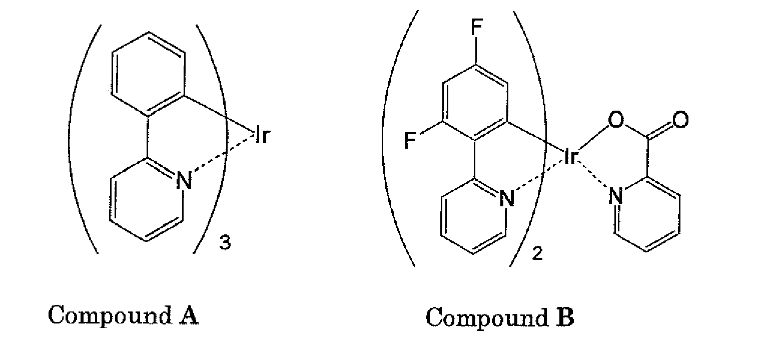 Although the above Compounds A and B are complexes having a bidentate chelate ligand, almost no complex having a tridentate chelate ligand similar to the above Compounds is known except Compound C below. (refer to Non-patent literature 3 below) 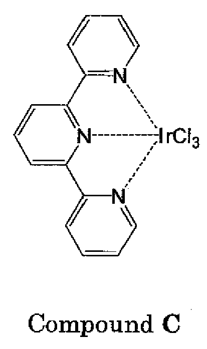 However, Compound C serves to emit reddish light having light emission wavelength of around 600 nm, without capability of serving to emit bluish light. Accordingly, a realization of a complex having a tridentate chelate ligand which serves to emit bluish light, if possible, has a possibility of new technology development.

Non-patent literature 1: D. F. O'Brien and M. A. Baldo et al. "improved energy transfer in electrophosphorescent devices" Applied Physics letters Vol.74 No.3, pp442-444, January 18, 1999
Non-patent literature 2: M.A. Baldo et al. "Very high-efficiency green organic light-emitting devices based on electrophosphorescence" Applied Physics letters Vol. 75 No.1, pp4-6, July 5, 1999
Non-patent literature 3: J-P. Collin et al., J. Am. Chem. Soc., 121,5009(1999)

### DISCLOSURE OF THE INVENTION

The present invention has been made to overcome the above problems and has an object of providing an organic EL device which emits blue light of high purity and of short wavelength with an enhanced efficiency of light emission, and an object of providing a metal-complex compound realizing it.

The inventors clarified a novel structural factor for enabling to emit blue light that employing a metal-complex compound with partial structure having a tridentate chelate ligand represented by a following general formula (I) enables to emit highly pure blue light of short wavelength and the present invention has been accomplished.
Namely, the present invention provides a metal-complex compound which comprises a tridentate chelate ligand having a partial structure represented by a following general formula (I).

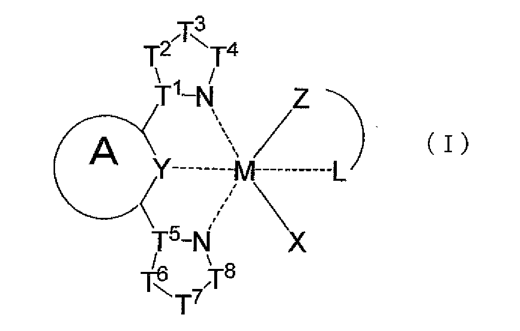

wherein M represents any one metal atom of Group 9 in Periodic Table;
L and Z each independently represent an organic group possessing any one atom of Groups 14 to 16 in Periodic Table respectively;
X represents a monovalent ligand having any one atom of Groups 14 to 17 in Periodic Table;
Y represents any one atom of Groups 14 to 16 in Periodic Table;
A represents a cycloalkane moiety having 5 to 20 carbon atoms and further may have a substituent, an aromatic hydrocarbon group having 6 to 20 carbon atoms and further may have a substituent or a heterocyclic group having 2 to 20 carbon atoms and further may have a substituent indicating that a circle enclosing the sign A shows a ring structure comprising Y;
T¹ to T⁸ each independently represents a carbon atom or a nitrogen atom on which a hydrogen atom, a cyano group, a nitro group, a halogen atom, an alkyl group having 1 to 12 carbon atoms and further may have a substituent, an alkylamino group having 1 to 12 carbon atoms and further may have a substituent, an arylamino group having 6 to 20 carbon atoms and further may have a substituent, an alkoxy group having 1 to 12 carbon atoms and further may have a substituent, an alkoxy halide group having 1 to 12 carbon atoms and further may have a substituent, an aryloxy group having 6 to 20 carbon atoms and further may have a substituent, an aromatic hydrocarbon group having 6 to 20 carbon atoms and further may have a substituent, a heterocyclic group having 3 to 20 carbon atoms and further may have a substituent, an alkyl halide group having 1 to 12 carbon atoms and further may have a substituent, an alkenyl group having 2 to 12 carbon atoms and further may have a substituent, an alkynyl group having 2 to 12 carbon atoms and further may have a substituent or a cycloalkyl groups having 3 to 20 carbon atoms and further may have a substituent may bond.
Further, the present invention provides an organic EL device which comprises at least one organic thin film layer sandwiched between a pair of electrodes consisting of an anode and a cathode, wherein the organic thin film layer comprises the above metal-complex compound, which emits light by applying an electric voltage between the pair of electrodes.

The present invention provides an organic EL device which emits blue light of high purity and of short wavelength with an enhanced efficiency of light emission, and also provides a metal-complex compound realizing the EL device.

### BRIEF DESCRIPTION OF THE DRAWINGS

FIG. 1 is a diagram showing an X-ray crystal structure analysis of Metal-Complex Compound 1;
FIG. 2 is a diagram showing an X-ray crystal structure analysis of Metal-Complex Compound 2;
FIG. 3 is a diagram showing an X-ray crystal structure analysis of Metal-Complex Compound 3;
FIG. 4 is a diagram showing an X-ray crystal structure analysis of Metal-Complex Compound 4.
FIG. 5 is a diagram showing an X-ray crystal structure analysis of Metal-Complex Compound 60;
FIG. 6 is a diagram showing a light emission spectrum of Metal-Complex Compound 1 at a room temperature;
FIG. 7 is a diagram showing a light emission spectrum of Metal-Complex Compound 2 at a room temperature;
FIG. 8 is a diagram showing a light emission spectrum of Metal-Complex Compound 3 at a room temperature;
FIG. 9 is a diagram showing a light emission spectrum of Metal-Complex Compound 4 at a room temperature;
FIG. 10 is a diagram showing a light emission spectrum of Metal-Complex Compound 47 at a room temperature;
FIG. 11 is a diagram showing a light emission spectrum of Metal-Complex Compound 48 at a room temperature;
FIG. 12 is a diagram showing a light emission spectrum of Metal-Complex Compound 49 at a room temperature;
FIG. 13 is a diagram showing a light emission spectrum of Metal-Complex Compound 60 at a room temperature;
FIG. 14 is a diagram showing a light emission spectrum of Metal-Complex Compound 61 at a low temperature;
FIG. 15 is a diagram showing a light emission spectrum of Metal-Complex Compound 1 at a low temperature;
FIG. 16 is a diagram showing a light emission spectrum of Metal-Complex Compound 2 at a low temperature.
FIG. 17 is a diagram showing a light emission spectrum of Metal-Complex Compound 3 at a low temperature;
FIG. 18 is a diagram showing a light emission spectrum of Metal-Complex Compound 4 at a low temperature;
FIG. 19 is a diagram showing a light emission spectrum of Metal-Complex Compound 47 at a low temperature;
FIG. 20 is a diagram showing a light emission spectrum of Metal-Complex Compound 48 at a low temperature;
FIG. 21 is a diagram showing a light emission spectrum of Metal-Complex Compound 49 at a low temperature;
FIG. 22 is a diagram showing a light emission spectrum of Metal-Complex Compound 60 at a low temperature.
FIG. 23 is a diagram showing a light emission spectrum of Metal-Complex Compound 61 at a low temperature;
FIG. 24 is a diagram showing an Ultraviolet Ray absorption spectrum of Metal-Complex Compound 1;
FIG. 25 is a diagram showing an Ultraviolet Ray absorption spectrum of Metal-Complex Compound 2;
FIG. 26 is a diagram showing an Ultraviolet Ray absorption spectrum of Metal-Complex Compound 3;
FIG. 27 is a diagram showing an Ultraviolet Ray absorption spectrum of Metal-Complex Compound 4;
FIG. 28 is a diagram showing an Ultraviolet Ray absorption spectrum of Metal-Complex Compound 47;
FIG. 29 is a diagram showing an Ultraviolet Ray absorption spectrum of Metal- Complex Compound 48;
FIG. 30 is a diagram showing an Ultraviolet Ray absorption spectrum of Metal-Complex Compound 49;
FIG. 31 is a diagram showing an Ultraviolet Ray absorption spectrum of Metal-Complex Compound 60; and
FIG. 32 is a diagram showing an Ultraviolet Ray absorption spectrum of Metal-Complex Compound 61.

### THE PREFERRED EMBODIMENT TO CARRY OUT THE INVENTION

The present invention provides a metal-complex compound having a partial structure represented by a following general formula (I):

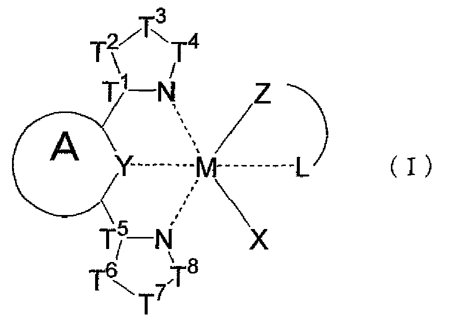

In the general formula (I), M represents any one metal atom of Group 9 in Periodic Table, and examples include Co (cobalt) atom, Rh (rhodium) atom and Ir (iridium) atom while Ir atom is preferable.
In the general formula (I), L and Z each independently represent an organic group possessing any one atom of Groups 14 to 16 in Periodic Table respectively.
Examples of the above atom of Groups 14 to 16 in Periodic Table possessed by L and Z include C (carbon) atom, N (nitrogen) atom, O (oxygen) atom, Si (silicon) atom, P (phosphorus) atom, S (sulfur) atom, Ge (germanium) atom, As (arsenic) atom, Se (selenium) atom and so on, while carbon atom, nitrogen atom and oxygen atom are preferable.
Further, the organic group represented by L and Z are preferably a hydrogen atom, a cyano group, a nitro group, a halogen atom, an alkyl group having 1 to 12 carbon atoms and further may have a substituent, an alkylamino group having 1 to 12 carbon atoms and further may have a substituent, an arylamino group having 6 to 20 carbon atoms and further may have a substituent, an alkoxy group having 1 to 12 carbon atoms and further may have a substituent, an alkoxy halide group having 1 to 12 carbon atoms and further may have a substituent, an aryloxy group having 6 to 20 carbon atoms and further may have a substituent, an aromatic hydrocarbon group having 6 to 20 carbon atoms and further may have a substituent, a heterocyclic group having 3 to 20 carbon atoms and further may have a substituent, an alkyl halide group having 1 to 12 carbon atoms and further may have a substituent, an alkenyl group having 2 to 12 carbon atoms and further may have a substituent, an alkynyl group having 2 to 12 carbon atoms and further may have a substituent or a cycloalkyl groups having 3 to 20 carbon atoms and further may have a substituent, etc.

Examples of the halogen atom include fluorine atom, chlorine atom, bromine atom, iodine atom and so on while fluorine atom is preferable.
Examples of the alkyl group include methyl group, ethyl group, propyl group, isopropyl group, n-butyl group, s-butyl group, isobutyl group, t-butyl group, n-pentyl group, n-hexyl group, n-heptyl group, n-octyl group, etc.
Examples of the aromatic hydrocarbon group include moieties of benzene, naphthalene, anthracene, phenanthrene, pyrene, biphenyl, terphenyl, fluoranthene, etc.
Examples of the heterocyclic group include moieties of imidazole, benzimidazole, pyrrole, furan, thiophene, benzothiophene, oxadiazoline, indoline, carbazole, pyridine, quinoline, isoquinoline, benzoquinone, pyrazoline, imidazolidine, piperidine, etc.
Examples of alkylamino group include a group formed by substituting a hydrogen atom of an amino group with the above alkyl group.
Examples of the arylamino group include a group formed by substituting a hydrogen atom of an amino group with the above aromatic hydrocarbon group.
The alkoxy group is expressed as -OY', wherein Y' represents the same as the foregoing examples about the above alkyl group.
Examples of the alkoxy halide group include a group formed by substituting a hydrogen atom of the above alkoxy group with the above halogen atom.
The aryloxy group is expressed as -OY", wherein Y" represents the same as the foregoing examples about the above aromatic hydrocarbon group.
Examples of the alkyl halide group include a group formed by substituting a hydrogen atom of the above alkyl group with the above halogen atom, preferably with a fluorine atom.
Examples of the alkenyl group include vinyl group, allyl group, 2-butenyl group, 3-pentenyl group, etc.
Examples of the alkynyl group include ethynyl group, methyl ethynyl group, etc.
Examples of the cycloalkyl group include cyclopropyl group, cyclobutyl group, cyclopentyl group, cyclohexyl group, etc.
Further, examples of the substituent for those groups include halogen atom, hydroxyl group, a substituted or unsubstituted amino group, nitro group, cyano group, a substituted or unsubstituted alkyl group, fluorinated alkyl group, a substituted or unsubstituted alkenyl group, a substituted or unsubstituted cycloalkyl group, a substituted or unsubstituted alkoxyl group, a substituted or unsubstituted heterocyclic group, a substituted or unsubstituted arylalkyl group, a substituted or unsubstituted aryloxy group, a substituted or unsubstituted alkoxycarbonyl group, carboxyl group, etc.

In the general formula (I), X represents a monovalent ligand possessing any one atom of Groups 14 to 17 in Periodic Table.
Examples of the atom of Groups 14 to 17 in Periodic Table and possessed by X include C (carbon) atom, N (nitrogen) atom, O (oxygen) atom, F (fluorine) atom, Si (silicon) atom, P (phosphorus) atom, S (sulfur) atom, Cl (chlorine) atom, Ge (germanium) atom, As (arsenic) atom, Se (selenium) atom, Br (bromine) atom, I (iodine) atom and so on, while carbon atom, nitrogen atom, chlorine atom, bromine atom and iodine atom are preferable.
Further, examples of the ligand represented by X include methoxy group, phenoxy group, cyano group, chlorine atom, bromine atom, iodine atom and so on, while cyano group, chlorine atom, bromine atom and iodine atom are preferable.
In the general formula (I), Y represents any one atom of Groups 14 to 16 in Periodic Table. Examples of the above atom of Groups 14 to 16 in Periodic Table expressed by Y include C (carbon) atom, N (nitrogen) atom, O (oxygen) atom, Si (silicon) atom, P (phosphorus) atom, S (sulfur) atom, Ge (germanium) atom, As (arsenic) atom, Se (selenium) atom and so on, while carbon atom, nitrogen atom and oxygen atom are preferable.
In the general formula (I), A represents a cycloalkane moiety having 6 to 20 carbon atoms and further may have a substituent, an aromatic hydrocarbon group having 6 to 20 carbon atoms and further may have a substituent or a heterocyclic group having 2 to 20 carbon atoms and further may have a substituent indicating that a circle enclosing the sign A shows a ring structure comprising Y.
Examples of the above moiety of cycloalkane include moieties of cyclopropane, cyclobutane, cyclopentane, cyclohexane, cycloheptane, etc.
Further, specific examples of the aromatic hydrocarbon group and the heterocyclic group include the same as described about the organic group represented by L and Z.
Furthermore, substituents for those groups are the same as the foregoing description.

In the general formula (I), T¹ to T⁸ each independently represents a carbon atom or a nitrogen atom on which a hydrogen atom, a cyano group, a nitro group, a halogen atom, an alkyl group having 1 to 12 carbon atoms and further may have a substituent, an alkylamino group having 1 to 12 carbon atoms and further may have a substituent, an arylamino group, having 6 to 20 carbon atoms and further may have a substituent, an alkoxy group having 1 to 12 carbon atoms and further may have a substituent, an alkoxy halide group having 1 to 12 carbon atoms and further may have a substituent, an aryloxy group having 6 to 20 carbon atoms and further may have a substituent, an aromatic hydrocarbon group having 6 to 20 carbon atoms and further may have a substituent, a heterocyclic group having 3 to 20 carbon atoms and further may have a substituent, an alkyl halide group having 1 to 12 carbon atoms and further may have a substituent, an alkenyl group having 2 to 12 carbon atoms and further may have a substituent, an alkenyl group having 2 to 12 carbon atoms and further may have a substituent or a cycloalkyl groups having 3 to 20 carbon atoms and further may have a substituent may bond. Specific examples of the above groups include the same as described about the organic group represented by L and Z.
Specific substituents of those groups are the same as the foregoing description.

In the general formula (I), the above tridentate chelate ligand is preferably any one of compounds shown by following general formulae (1) to (3), and further preferably any one of compounds shown by following general formulae (4) to (15).

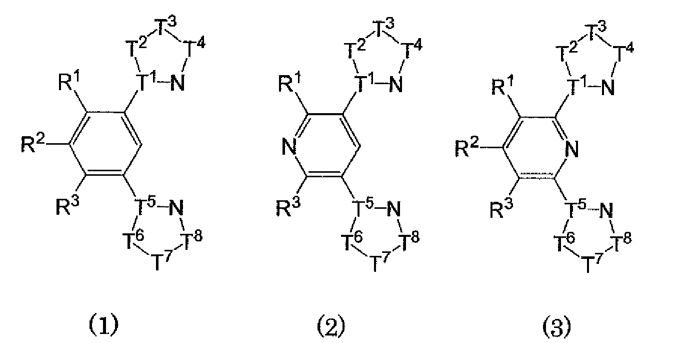

In the general formulae (1) to (3), T¹ to T⁸ are the same as the foregoing description; and R¹ to R³ each independently represents a hydrogen atom, a cyano group, a nitro group, a halogen atom, an alkyl group having 1 to 12 carbon atoms and further may have a substituent, an alkylamino group having 1 to 12 carbon atoms and further may have a substituent, an arylamino group having 6 to 20 carbon atoms and further may have a substituent, an alkoxy group having 1 to 12 carbon atoms and further may have a substituent, an alkoxy halide group having 1 to 12 carbon atoms and further may have a substituent, an aryloxy group having 6 to 20 carbon atoms and further may have a substituent, an aromatic hydrocarbon group having 6 to 20 carbon atoms and further may have a substituent, a heterocyclic group having 3 to 20 carbon atoms and further may have a substituent, an alkyl halide group having 1 to 12 carbon atoms and further may have a substituent, an alkenyl group having 2 to 12 carbon atoms and further may have a substituent, an alkynyl group having 2 to 12 carbon atoms and further may have a substituent or a cycloalkyl groups having 3 to 20 carbon atoms and further may have a substituent.
Specific examples and substituents of those groups include the same as described about the organic group represented by L and Z.

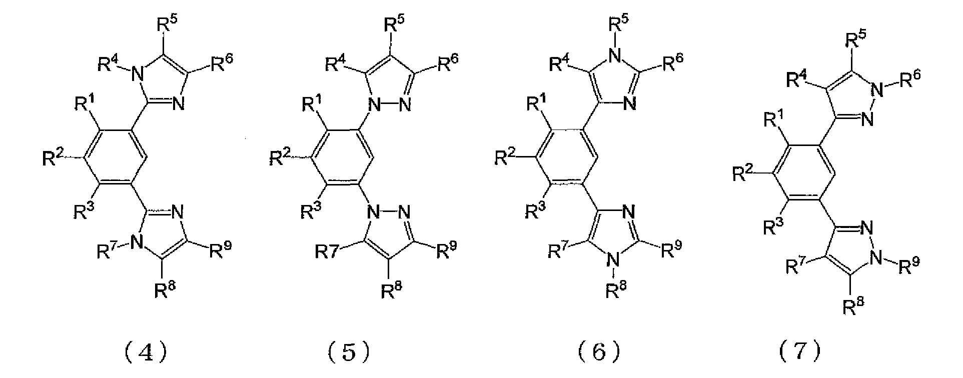

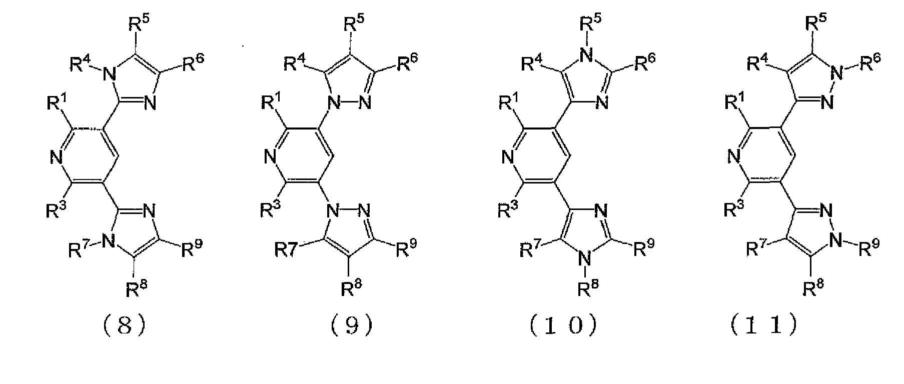

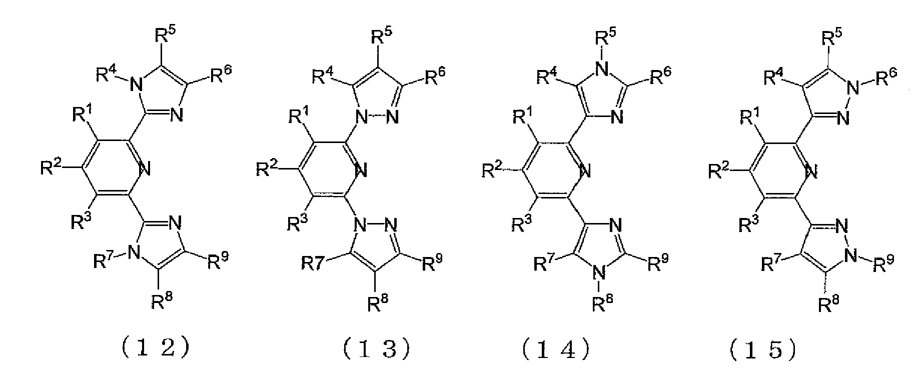

In the general formulae (4) to (15), R¹ to R⁹ each independently represents the same as described about the above R¹ to R³, and specific examples and their substituents are the same as described about the above R¹ to R³. However, the above R¹ to R³ never bonds each other.

In the general formula (I), the ligand formed by L-Z is preferably a compound expressed by any one of following general formulae (16) to (24):

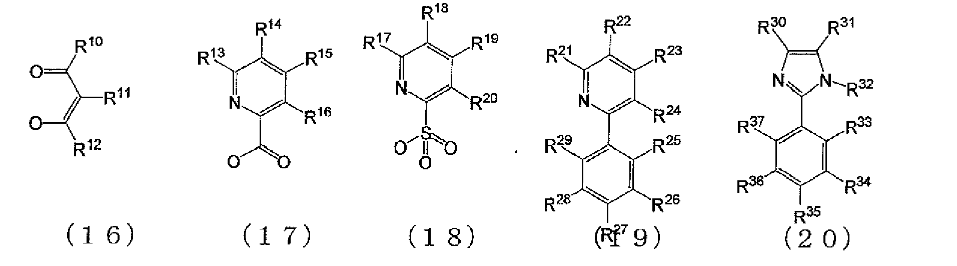

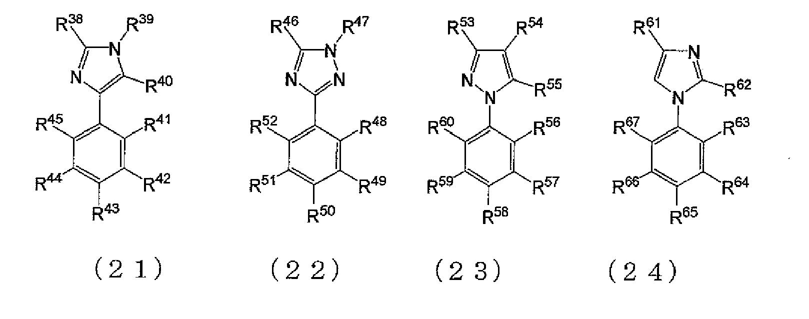

In the general formulae (16) to (24), R¹⁰ to R⁶⁷ each independently represents the same as described about the above R¹ to R³, and specific examples and their substituents are the same as described about the above R¹ to R³.

It is preferable that the metal complex compound of the present invention is preferably shown by any one among following general formulae (I-1) to (I-8) and general formulae (I-9) to (I-12):

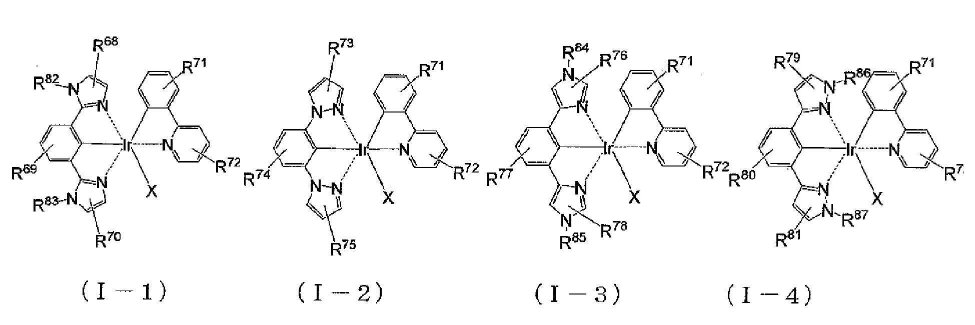

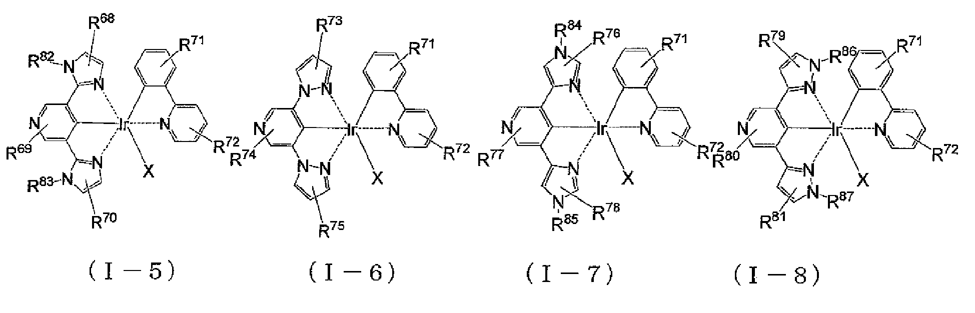

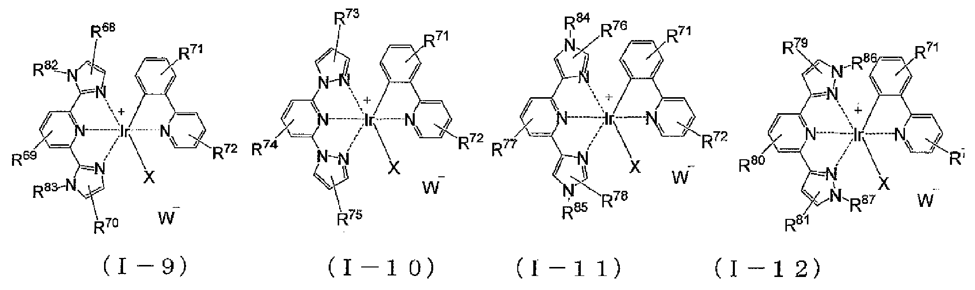

In the general formulae (I-1) to (I-12), R⁶⁸ to R⁸⁷ each independently represents the same as described about the above R¹ to R³, and specific examples and their substituents are the same as described about the above R¹ to R³. Further, the number of R⁶⁸ to R⁸⁷ may be plural and when any R⁶⁸ to R⁸⁷ exists plurally, they may be the same with or different from each other.
In the general formulae (I-9) to (I-12), W⁻ is an anion having charge of -1 comprising at least one kind of metal atom among Groups 13 to 16 in Periodic Table and examples include PF₆⁻ ClO₄⁻, SbF₆⁻, OTf⁻, OTs^{-,} BF₄⁻, BPh₄⁻, B(C₆F₅)₄⁻, and so on, while PF₆⁻ is preferable.

Specific examples of the metal-complex compound of the present invention are as follows, however, the present invention is not limited to these typical compounds.

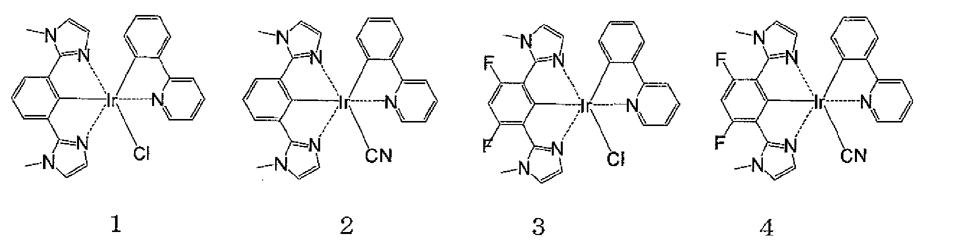

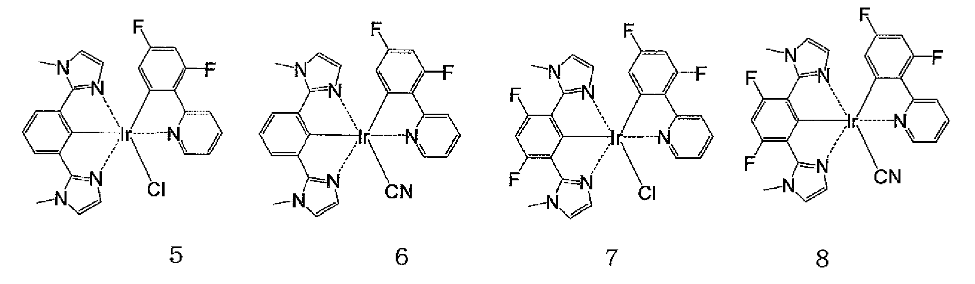

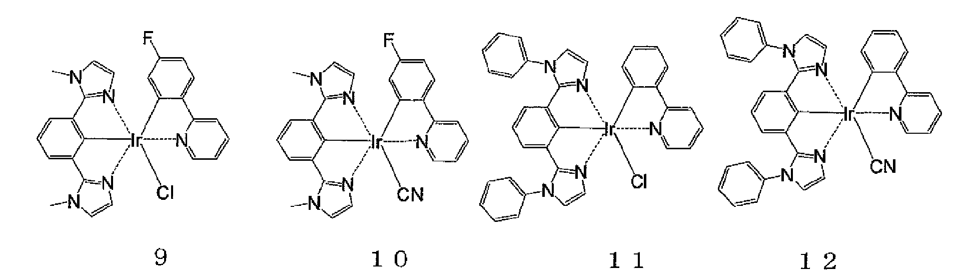

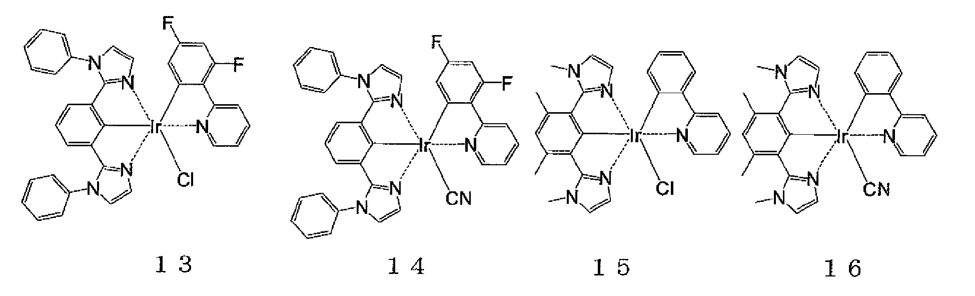

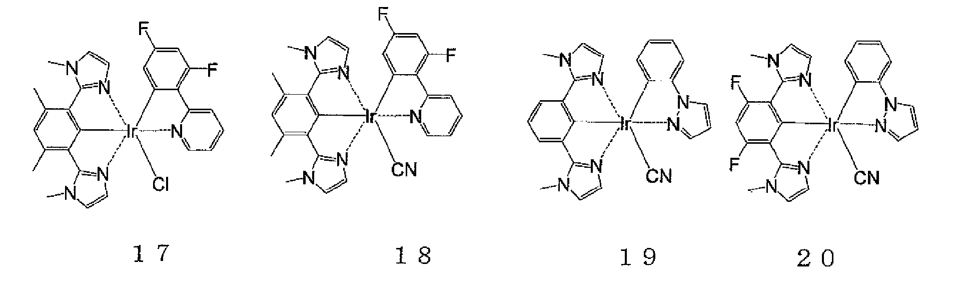

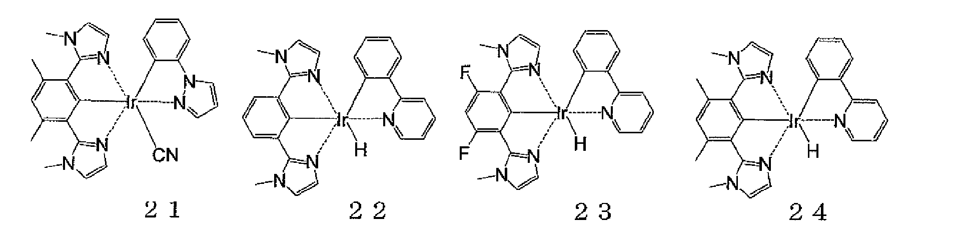

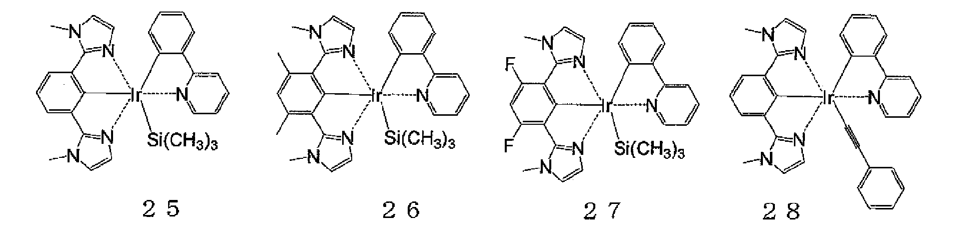

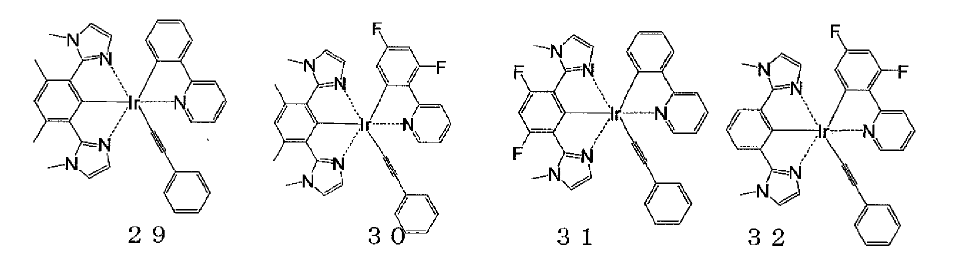

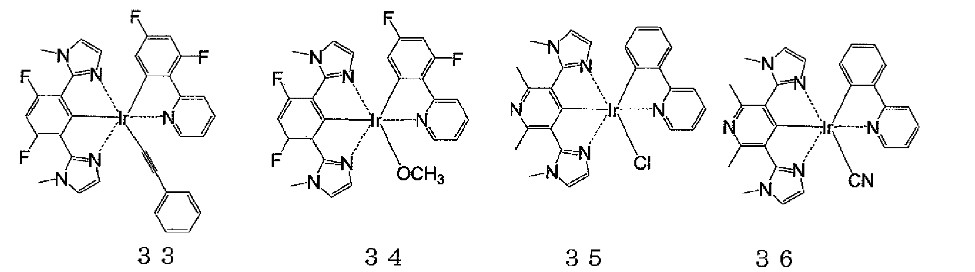

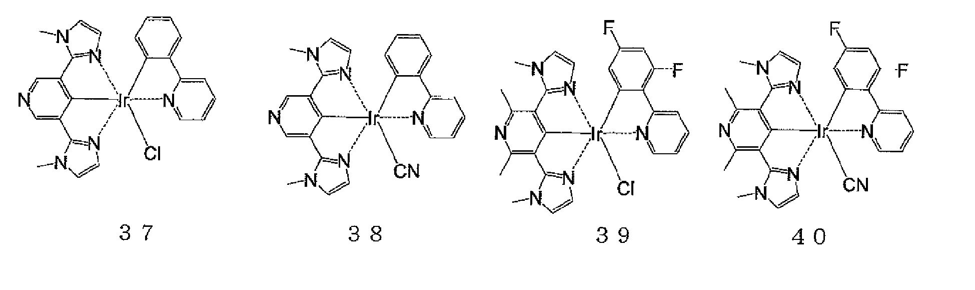

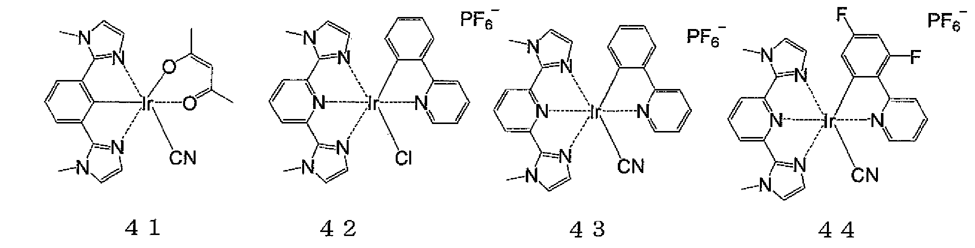

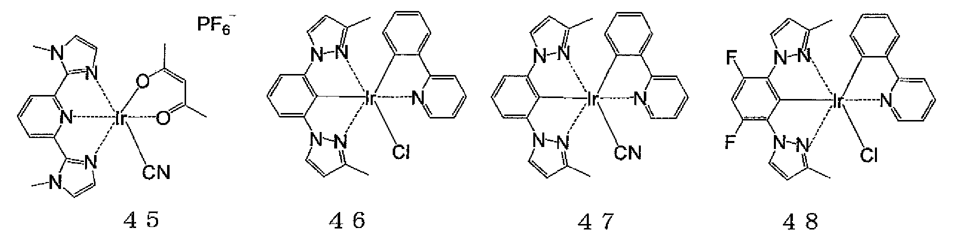

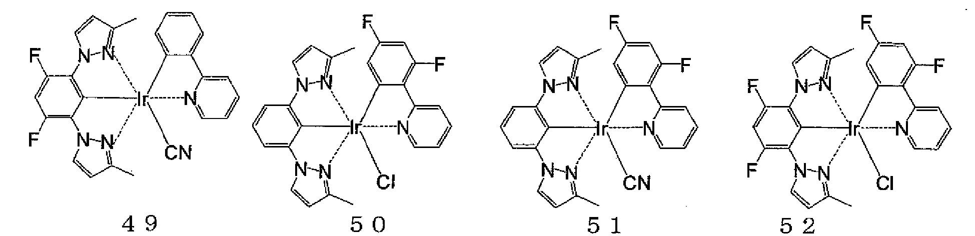

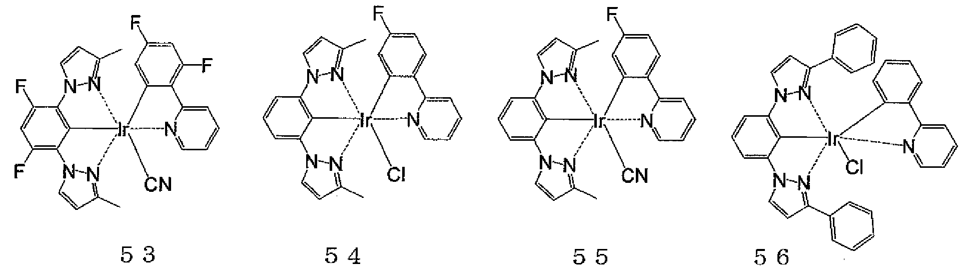

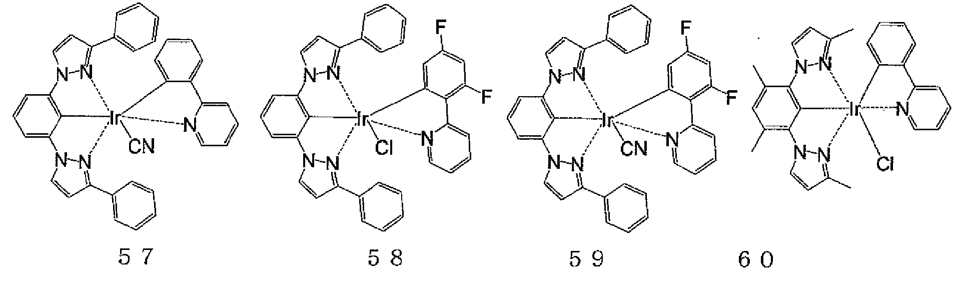

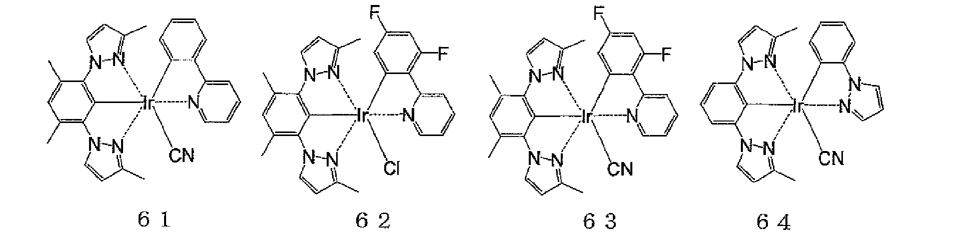

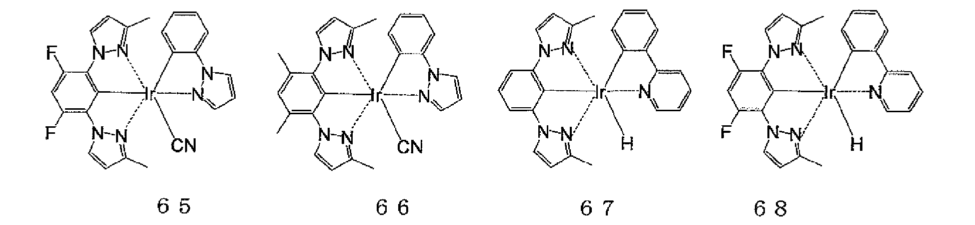

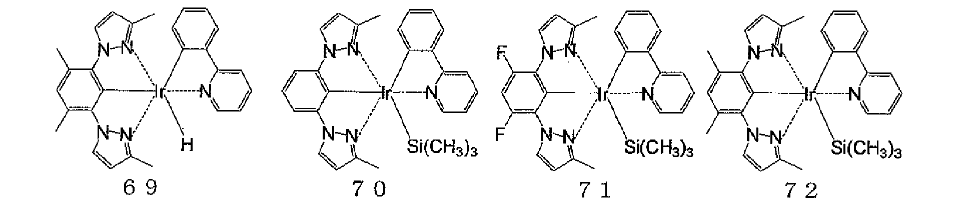

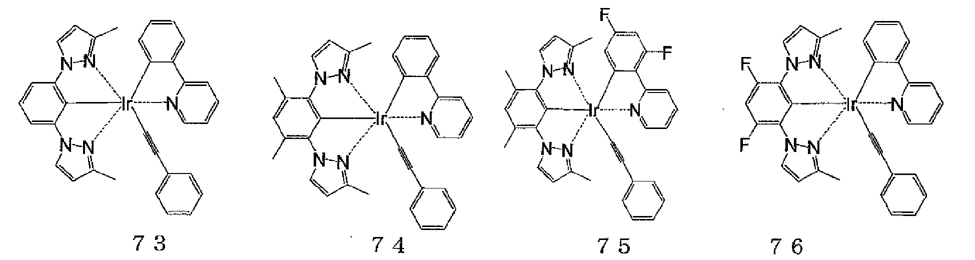

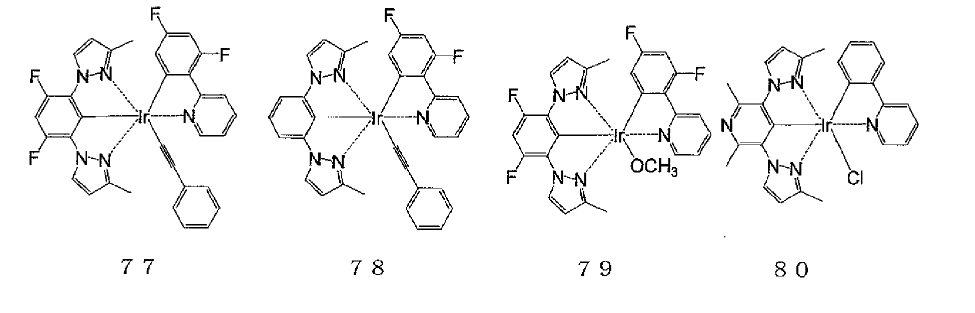

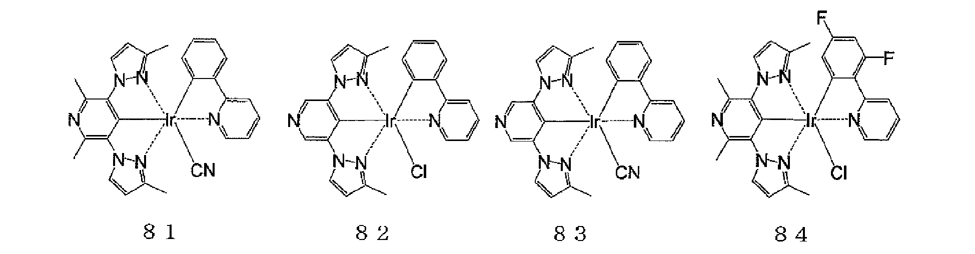

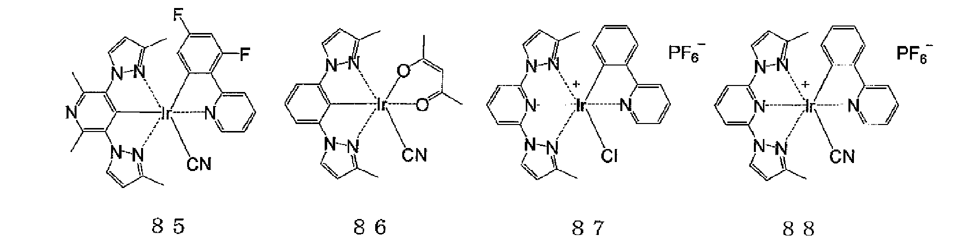

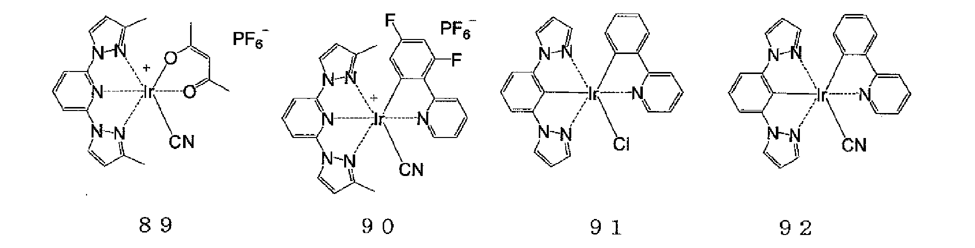

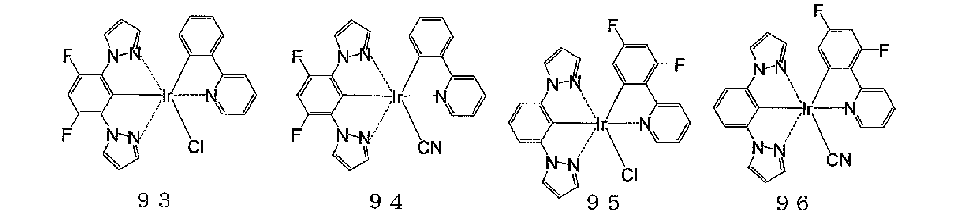

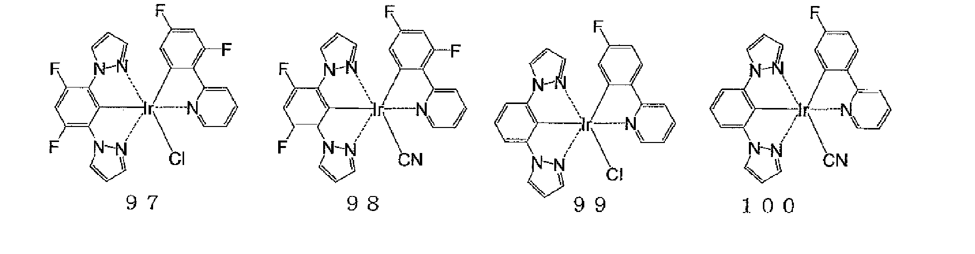

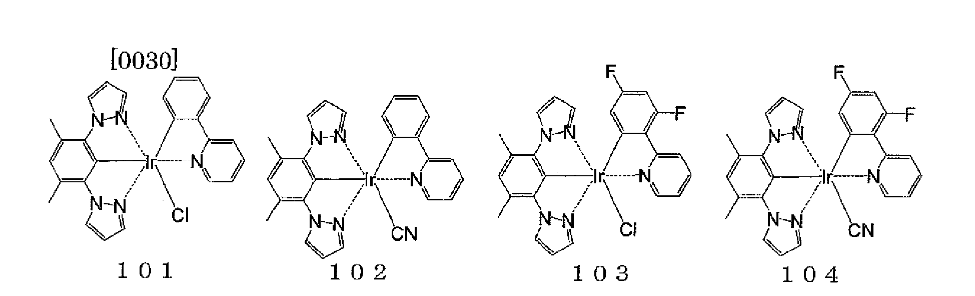

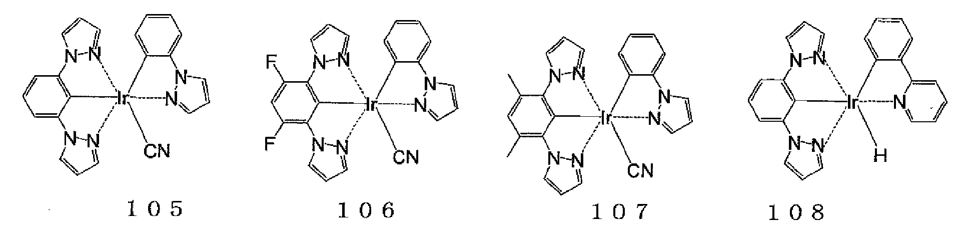

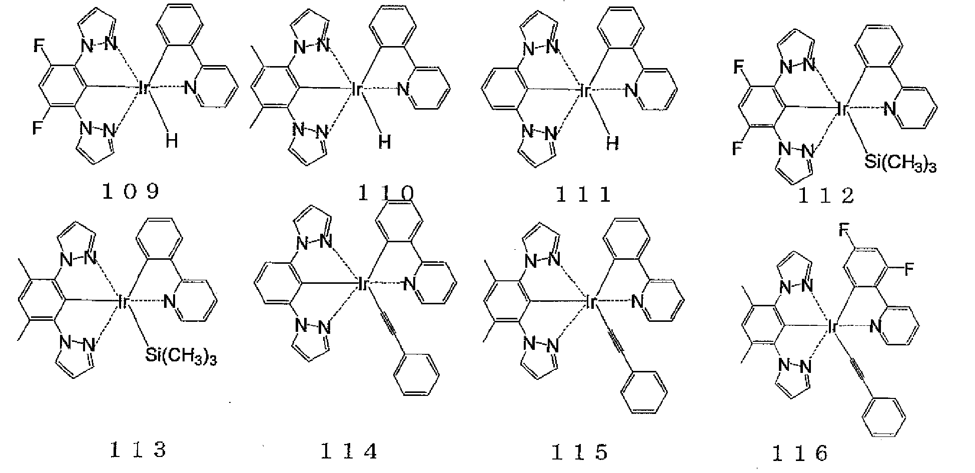

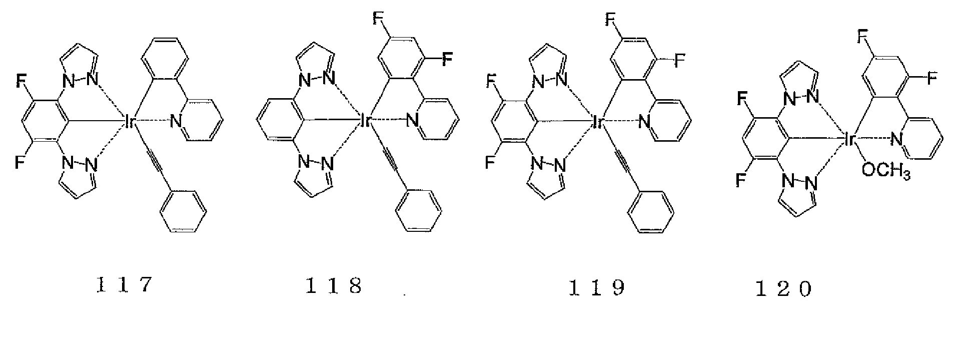

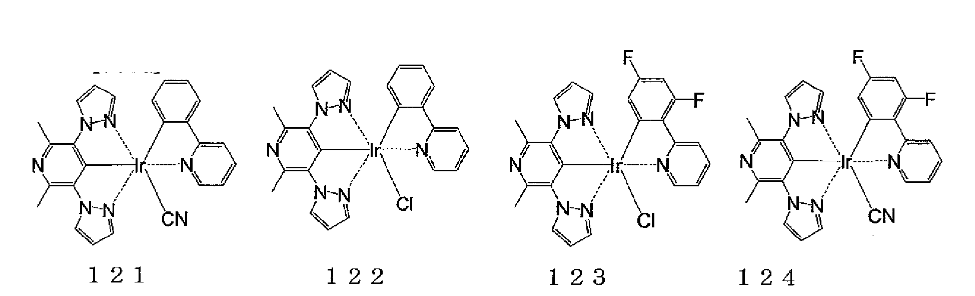

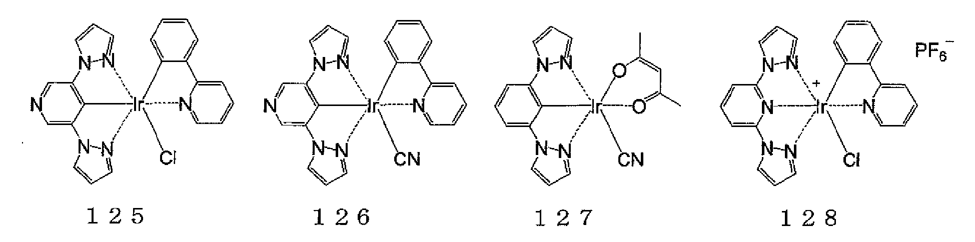

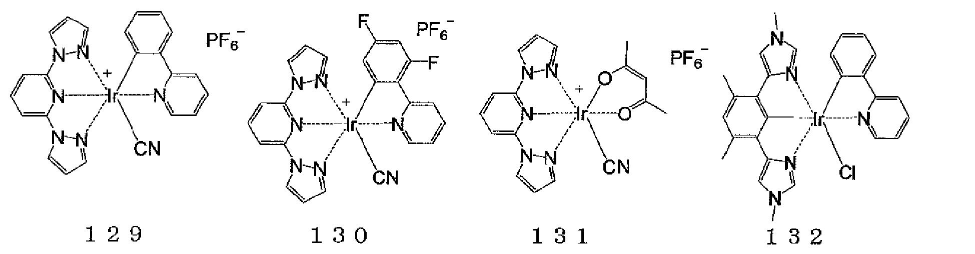

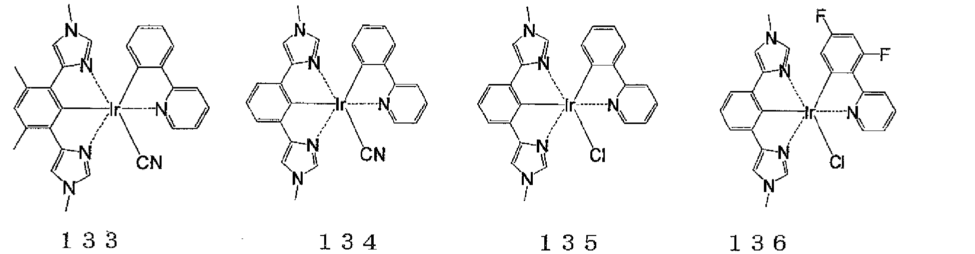

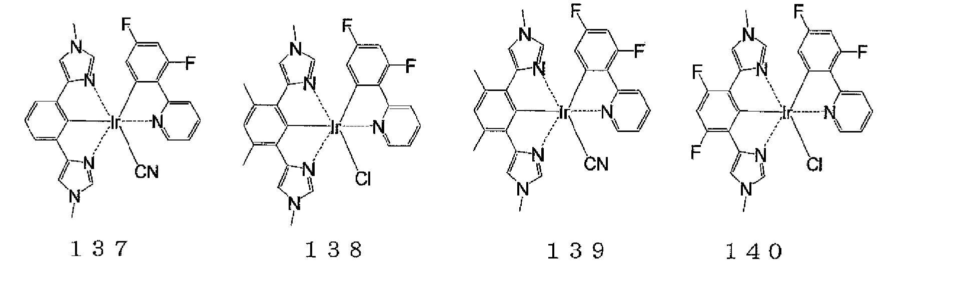

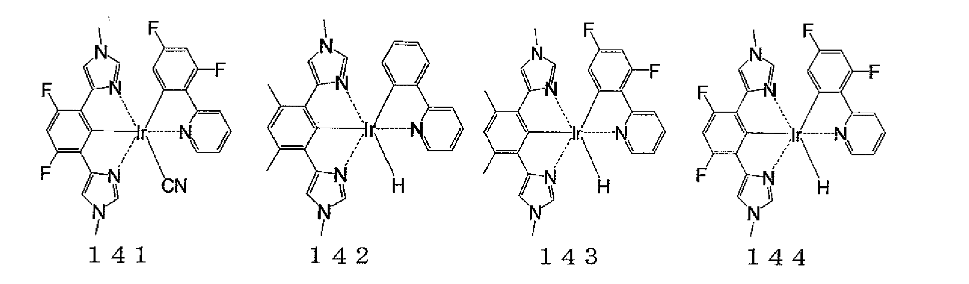

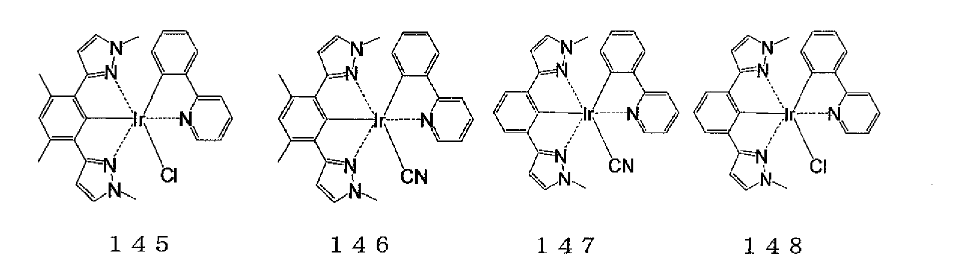

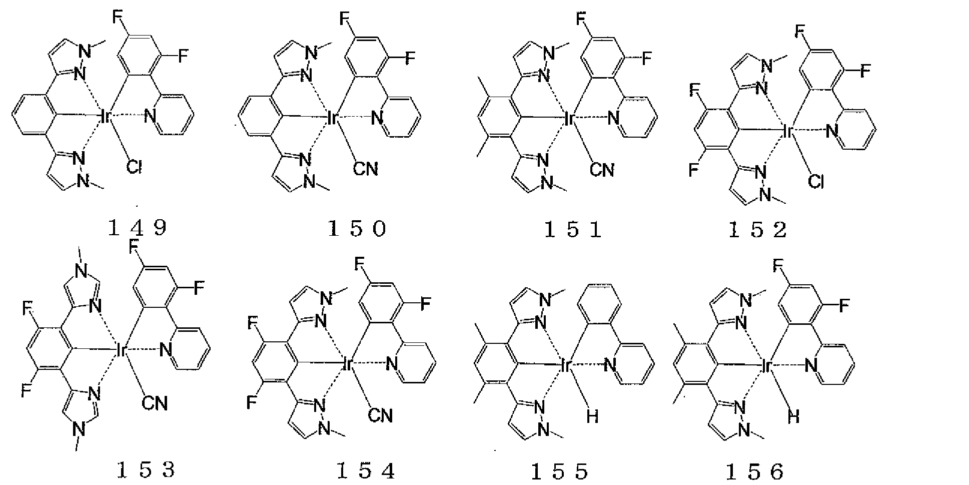

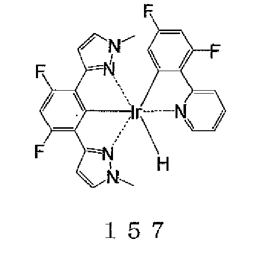

The present invention provides an organic EL device which comprises one or more organic thin film layers having at least one light emitting layer sandwiched between a pair of electrodes consisting of an anode and a cathode, wherein the organic thin film layer comprises the foregoing metal-complex compound, which emits light by applying an electric voltage between the pair of electrodes.
It is preferable for the organic EL device of the present invention that the light emitting layer comprises the metal-complex compound of the present invention, and that it comprises the metal-complex compound of the present invention in an amount of 1 to 30 % by weight of total weight of the light emitting layer.
Further, the light emitting layer is usually formed to a thin film by means of vacuum vapor deposition process or coating process, however, it is preferable that the layer comprising the metal-complex compound of the present invention is formed into film by coating process because it simplifies the production process.

The organic EL device of the present invention is fabricated by laminating one or more organic layers between a pair of electrodes and examples of the construction include (i) an anode / a light emitting layer / a cathode; (ii) an anode / a hole injecting and transporting layer / a light emitting layer / an electron injecting and transporting layer / a cathode; (iii) an anode / a hole injecting and transporting layer / a light emitting layer / a cathode; and (iV) an anode / a light emitting layer / an electron injecting and transporting layer / a cathode.
The compound in the present invention may be used in any of the foregoing organic layer, or may be doped into other hole transporting materials, light emitting materials and electron transporting materials. The process for forming the layers in the organic EL device of the present invention is not particularly limited. In addition to the vapor deposition process, after dissolving the light emitting composition of the present invention or after dissolving the compound forming the composition, the resultant solution may be formed into a light emitting medium or a light emitting layer by means of various wet processes. Namely, they may be formed in accordance with a conventional coating process such as the dipping process, the spin coating process, the casting process, the bar coating process and the roller coating process or with an ink-jet process. The thickness of each layer in the organic thin film layer in the organic EL device of the present invention is not particularly limited. In general, an excessively thin layer tends to have defects such as pin holes, and an excessively thick layer requires a high applied voltage resulting in decreasing the efficiency. Therefore, a thickness within the range of several nanometers to 1 µm is preferable.

Examples of the solvent used for preparing light emitting solution for the light emitting layer include, halogen-based hydrocarbon solvent such as dichloromethane, dichloroethane, chloroform, tetrachloromethane, tetrachloroethane, trichloroethane, chlorobenzene, dichlorobenzene, chlorotoluene, etc.; ether-based solvent such as dibutylether, tetrahydrofuran, dioxane, anisole, etc.; alcohol-based solvent such as methanol, ethanol, propanol, butanol, pentanol, hexanol, cyclohexanol, methylcellosolve, ethylcellosolve, ethylene glycol, etc.; hydrocarbon-based solvent such as benzene, toluene, xylene, ethyl benzene, hexane, octane, decane, etc.; ester-based solvent such as ethyl acetate, butyl acetate, amyl acetate, etc. Among those, halogen-based hydrocarbon solvent, hydrocarbon-based solvent and ether-based solvent are preferable. Further, the solvent may be used alone, or in combination of two or more kind thereof. Additionally, the employable solvent is not limited to the above examples. Still further, a dopant may be optionally dissolved in advance, into the solution for the light emitting layer.

Electron injecting and transporting material employed for the present invention is not particularly specified and any compound usually employed as electron injecting and transporting material may be employable. Examples include oxadiazole derivatives such as 2-(4-biphenylyl)-5-(4-t-butylphenyl)-1,3,4-oxadiazole, bis{2-(4-t-butylphenyl)-1,3,4-oxadiazole}-m-phenylene, triazole derivatives and quinolinol-based metal-complex. As an inorganic compound for an electron injecting and transporting layer it is preferable to employ an insulating material or a semiconductor.

The electron injecting and transporting layer effectively prevents leak in the electric current and improves the electron injecting capability.
It is preferable that at least one metal compound selected from the group consisting of alkali metal chalcogenides, alkaline earth metal chalcogenides, alkali metal halides and alkaline earth metal halides is used as the insulating material. It is preferable that the electron injecting and transporting layer is constituted with the above alkali metal chalcogenide since the electron injecting property can be improved.
Preferable examples of the alkali metal chalcogenide include Li₂O, Na₂S and Na₂Se. Preferable examples of the alkaline earth metal chalcogenide include CaO, BaO, SrO, BeO, BaS and CaSe. Preferable examples of the alkali metal halide include LiF, NaF, KF, LiCl, KCl and NaCl. Preferable examples of the alkaline earth metal halide include fluorides such as CaF₂, BaF₂, SrF₂, MgF₂ and BeF₂ and halides other than the fluorides.

Examples of the semiconductor constituting the electron injecting and transporting layer include oxides, nitrides or oxynitrides containing at least one element selected from Ba, Ca, Sr, Yb, Al, Ga, In, Li, Na, Cd, Mg, Si, Ta, Sb and Zn, which are used singly or in combination of two or more. It is preferable that the inorganic compound constituting the electron injecting and transporting layer is in the form of a fine crystalline or amorphous insulating thin film. When the electron injecting and transporting layer is constituted with the above insulating thin film, a more uniform thin film can be formed and defective pixels such as dark spots can be decreased. Examples of the inorganic compound include the alkali metal chalcogenides, the alkaline earth metal chalcogenides, the alkali metal halides and the alkaline earth metal halides which are described above.

In the present invention, a reductive dopant with a work function of 2.9 eV or smaller may be added in the electron injecting and transporting layer. The reductive dopant used in the present invention is defined as a substance which reduces the electron transporting compound. Accordingly, various substances having a reductive capability are employable and examples include at least one substance selected from alkali metals, alkaline earth metals, rare earth metals, alkali metal oxides, alkali metal halides, alkaline earth metal oxides, alkaline earth metal halides, rare earth metal oxides, rare earth metal halides, alkali metallic complexes, alkaline earth metallic complexes, and rare earth metallic complexes.

Examples of the preferable reductive dopant include at least one alkali metal selected from a group consisting of Na (the work function: 2.36 eV), K (the work function: 2.28 eV), Rb (the work function: 2.16 eV) and Cs (the work function: 1.95 eV) or at least one alkaline earth metals selected from a group consisting of Ca (the work function: 2.9 eV), Sr (the work function: 2.0 to 2.5 eV) and Ba (the work function: 2.52 eV); whose work function of 2.9 eV or smaller is particularly preferable. Among those, more preferable reductive dopants include at least one kind selected from the group consisting of K, Rb and Cs, the latter Rb or Cs being farther more preferable and the last Cs being the most preferable. Those alkali metals have particularly high reducing capability, and only an addition of relatively small amount of them into an electron injection zone enables to expect both improvement of luminance and lifetime extension of the organic EL device.
Further, with regard to the reductive dopant with work function of 2.9 eV or smaller, a combination of two or more kinds of the alkali metal is also preferable, and particularly, combinations containing Cs, for example, combinations of Cs and Na, Cs and K, Cs and Rb, Cs and Na and K are preferable. Containing Cs in combination enables to reveal reducing capability effectively, and the addition into the electron injection region expects both improvement of luminance and lifetime extension of the organic EL device.

The anode in the organic EL device covers a role of injecting holes into a hole injecting and transporting layer or into a light emitting layer, and it is effective that the anode has a work function of 4.5 eV or greater. Specific examples of the material for the anode include indium tin oxide (ITO) alloy, tin oxide (NESA), gold, silver, platinum, copper, etc. With regard to the cathode, its material preferably has a small work function with the aim of injecting electrons into an electron injecting and transporting layer and/or into a light emitting layer.
Further in the organic EL device, a hole injecting (transporting) layer may be disposed over the anode. Various organic compounds and polymers usually used for the organic EL device, for example, which are described in Japanese Unexamined Patent Application Laid-Open Nos. Shou 63-295695 and Hei 2-191694 may be employed as the hole injecting and transporting layer. Examples include aromatic tertiary amine, hydrazone derivative, carbazole derivatives, triazole derivatives, imidazole derivatives or polyvinylcarbazole, polyethylenedioxythiophene polystyrene sulfonic acid (PEDOT / PSS), etc.

Although materials for the cathode of the organic EL device are not particularly specified, examples include indium, aluminum, magnesium, magnesium-indium alloy, magnesium-aluminum alloy, aluminum-lithium alloy, aluminum-scandium-lithium alloy, magnesium-silver alloy, etc.

### EXAMPLE

The present invention shall be explained below in further details with reference to examples, but the present invention shall by no means be restricted by the following examples.

### Synthesis Example 1 (Synthesis of Metal-Complex Compound 1)

The route for synthesis of the above Metal-Complex Compound 1 and Metal-Complex Compound 2 below is illustrated as follows:

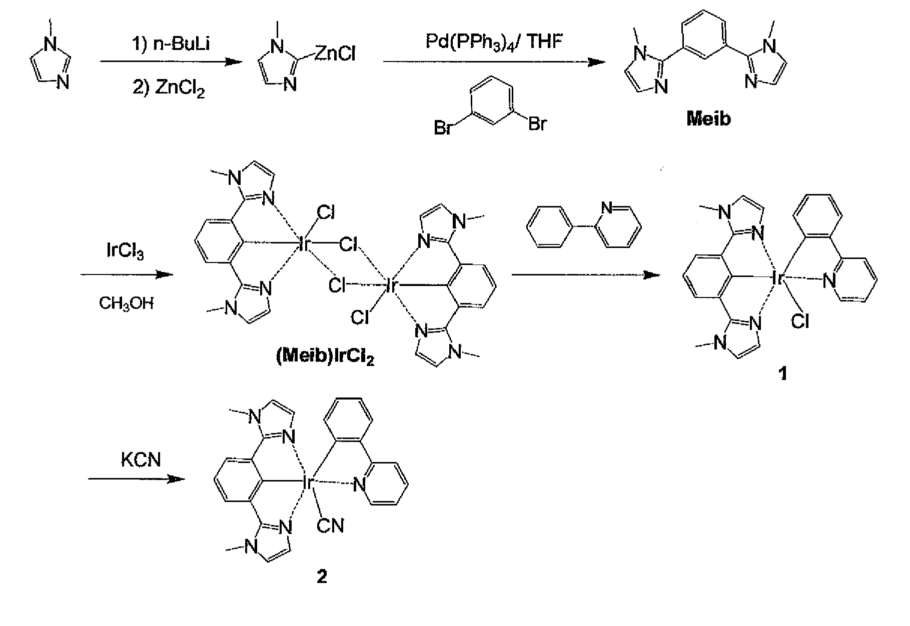

### (1) Synthesis of Meib

Replacing the air among a three-neck flask having a capacity of 100 milliliter with argon gas and after dissolving N-methylimidazole in an amount of 38 millimole (3.1 g) into dehydrated tetrahydrofuran (THF) in an amount of 50 milliliter, the resultant solution was cooled down to a temperature of -70 °C, followed by slowly adding t-butyllithium (2.66 M) in an amount of 44 millimole (16.5 milliliter). After stirring the solution for 15 minutes, anhydrous zinc chloride in an amount of 44 millimole (6.0 g) and dehydrated THF in an amount of 30 milliliter were added and stirred and then, the temperature of the solution was returned to a room temperature. Adding tetrakistriphenylphosphinepalladium in an amount of 0.252 millimole (0.29 g) and 1,3-dibromobenzene in an amount of 12.6 millimole (3.0 g) together with dehydrated THF in an amount of 20 milliliter into the solution, it was refluxed for one hour and a half. After cooling down the mixed solution, anhydrous zinc chloride in an amount of 76 millimole (10.4 g) was added further, the resultant solution was refluxed for 16 hours. Placing the reactant into a 1000 milliliter of pure water dissolving ethylenediamine tetraacetic acid tetrasodium salt anhydride in an amount of 236 millimole (88 g), adjusted pH to 8 with the use of 10 % sodium carbonate aqueous solution. Extracting a product from the solution with a use of methylene chloride, and after dehydrating the product with a use of sodium sulfate, the solution was filtered and the filtrate was concentrated. Carrying out a silica column purification (acetone / methanol= 9/1), a white crystal of the aimed substance Meib in an amount of 1.99 g (the yield: 44 %) was obtained.

### (2) Synthesis of (Meib) IrCl₂

Placing iridium chloride trihydrate in an amount of 1.42 millimole (0.5 g) and 2-ethoxyethanol in an amount of 50 milliliter into an eggplant flask having a capacity of 100 milliliter and dissolving them, Meib in an amount of 1.70 millimole (0.406 g) was added. After refluxing it for 6 hours, the resultant solution was filtered and dried and then, a yellow crystal of the aimed substance in an amount of 0.603 g (the yield: 84 %) was obtained.

### (3) Synthesis of Metal-Complex Compound 1

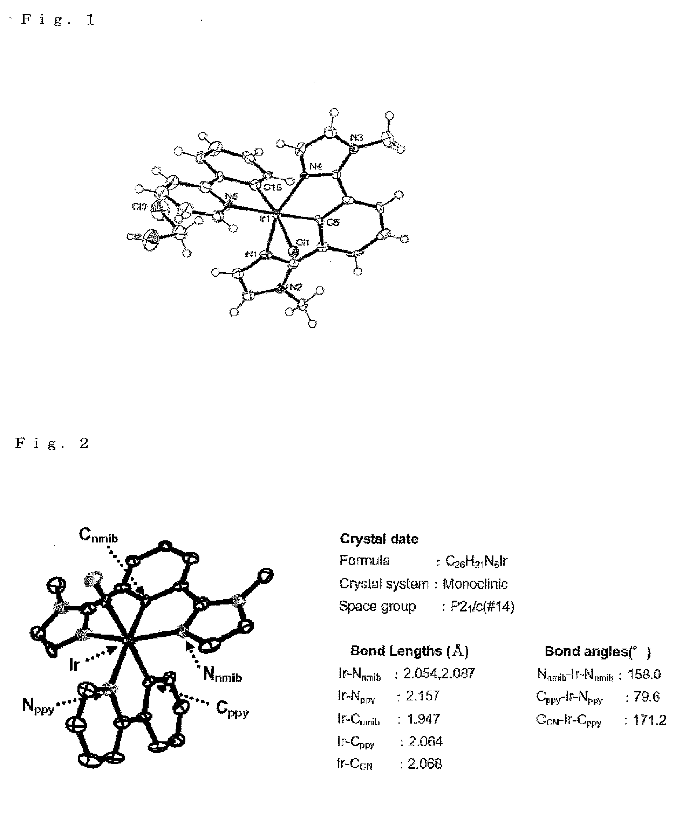

Into an eggplant flask having a capacity of 100 milliliter, **(Meib)** IrCl₂ in an amount of 0.782 millimole (0.783 g), 2-phenylpyridine in an amount of 87 millimole (0.291 g) and ethylene glycol in an amount of 80 milliliter were placed, and the resultant solution was refluxed with heating being exposed with microwave irradiation for 1 minute in 3 times under an atmospheric nitrogen gas flow. The reacted solution was cooled by being left standing and then, pouring pure water, a supernatant liquid was collected by means of a centrifugal separator. Adding saturated sodium chloride solution into the supernatant liquid, a generated precipitation was separated by filtration and washed with uses of hexane and diethyl ether. Carrying out a silica column purification (dichloro-methane / methanol= 25/1), a yellow crystal of the aimed substance in an amount of 0.453 g (the yield: 47 %) was obtained.
It was confirmed in accordance with a measurement of light emission spectrum about Metal-Complex Compound 1 obtained, that λₑₘ= 488 nanometers (excitation wavelength: 355 nanometers).
Further, the result of conducting X-ray crystal structure analysis (apparatus: Rigaku PAXIS-RAPID Imaging Plate Diffractometer) about Metal-Complex Compound 1 is shown in FIG. 1.

### Synthesis Example 2 (Synthesis of Metal-Complex Compound 2)

Into an eggplant flask having a capacity of 50 milliliter, Metal-Complex Compound 1 in an amount of 0.081 millimole (0.05 g), potassium cyanide in an amount of 4.0 millimole (0.263 g) and ethylene glycol in an amount of 50 milliliter were placed, and the resultant solution was refluxed with heating being exposed with microwave irradiation for 1 minute in 3 times under an atmospheric nitrogen gas flow. After cooling the reacted solution by leaving it standing, a saturated sodium chloride solution in an amount of 200 milliliter was added and the precipitate was collected by filtration.
Washing the precipitate with uses of hexane and diethyl ether, a silica column purification (dichloro-methane / methanol= 25/1) was carried out and a yellow crystal of the aimed substance in an amount of 0.332 g (the yield: 67 %) was obtained.
It was confirmed in accordance with a measurement of light emission spectrum about Metal-Complex Compound 2 obtained, that λₑₘ= 473 nanometers (excitation wavelength: 355 nanometers).
Further, the result of conducting X-ray crystal structure analysis about Metal-Complex Compound 2 is shown in FIG. 2.

### Synthesis Example 3 (Synthesis of Metal-Complex Compound 3)

The route for synthesis of the above Metal-Complex Compound 3 and Metal-Complex Compound 4 below is illustrated as follows:

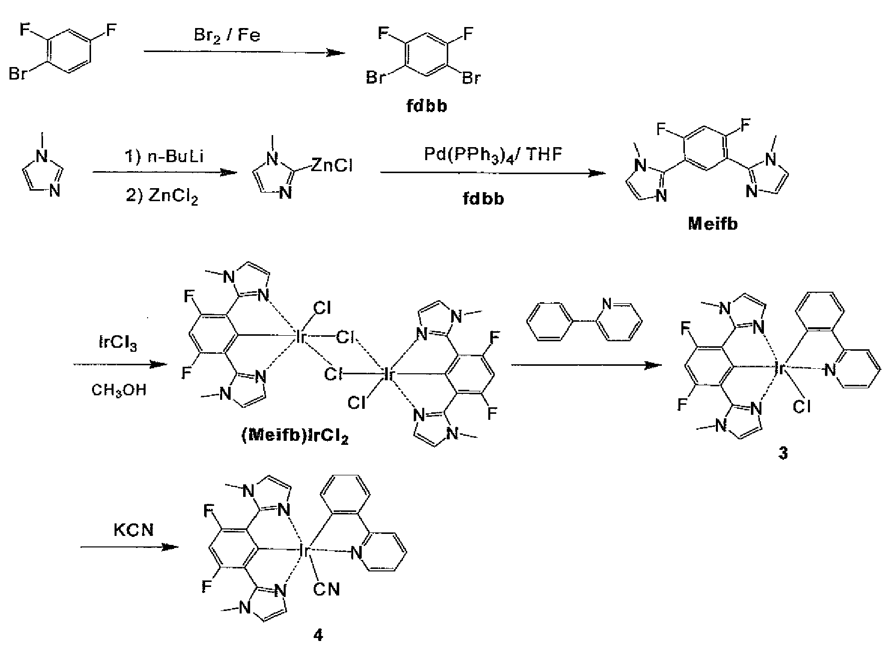

### (1) Synthesis of fdbb

Into a three-neck flask having a capacity of 100 milliliter, 1-bromo-2,4-difluorobenzene in an amount of 129 millimole (14.6 milliliter) was placed and heated up to a temperature of 60 °C. Then, after adding 0.45 g of iron, 175 millimole (9 milliliter) of bromine was dripped into the flask spending 3 hours. After completing the drip, the resultant solution was stirred at a temperature of 65 °C for 2 hours and a half. The reacted solution was cast into cold sodium hydroxide aqueous solution and the resultant solution was extracted with a use of hexane. An organic layer was washed with uses of pure water and saturated sodium chloride solution, and after dehydrating with anhydrous sodium sulfate, solvents were removed. Purifying the resultant product in accordance with a reduced-pressure distillation (38 °C / 3 Torr), 17.6 g (the yield: 50 %) of an aimed substance was obtained.

### (2) Synthesis of Meifb

Replacing the air among a three-neck flask having a capacity of 100 milliliter with argon gas and after dissolving 57 millimole (4.65 g) of N-methylimidazole into 75 milliliter of dehydrated THF, the resultant solution was cooled down to a temperature of -70 °C, followed by slowly adding n-butyllithium (2.66 M) in an amount of 64 millimole (24 milliliter). After stirring the solution for 15 minutes, anhydrous zinc chloride in an amount of 66 millimole (9.0 g) and dehydrated THF in an amount of 45 milliliter were added and stirred and then, the temperature of the solution was returned to a room temperature. Adding 1,1'-bisdiphenylphosphinoferrocenedichloropalladium in an amount of 0.378 millimole (0.309 g) and fdbb in an amount of 18.9 millimole (5.16 g) together with dehydrated THF in an amount of 30 milliliter into the solution, it was refluxed for 1 hour and a half. After cooling down the mixed solution, anhydrous zinc chloride in an amount of 114 millimole (15.6 g) was added further, and the resultant solution was refluxed for 20 hours.
Placing the reactant into 1500 milliliter of pure water dissolving ethylenediaminetetraacetic acid tetrasodium salt anhydride in an amount of 354 millimole (132 g), adjusted pH to 8 with the use of 10 % sodium carbonate aqueous solution. Extracting a product from the solution with a use of methylene chloride, and after dehydrating the product with a use of sodium sulfate, the solution was filtered and the filtrate was concentrated. Carrying out a silica column purification (acetone), a white crystal of the aimed substance Meifb in an amount of 1.02 g (the yield: 19 %) was obtained.

### (2) Synthesis of (Meifb) IrCl₂

Placing iridium chloride trihydrate in an amount of 1.42 millimole (0.5 g) and 2-ethoxyethanol in an amount of 50 milliliter into an eggplant flask having a capacity of 100 milliliter and dissolving them, Meifb in an amount of 1.70 millimole (0.466 g) was added. After refluxing it for 6 hours, the resultant solution was filtered and dried and then, a yellow crystal of the aimed substance in an amount of 0.575 g (the yield: 74 %) was obtained.

### (3) Synthesis of Metal-Complex Compound 3

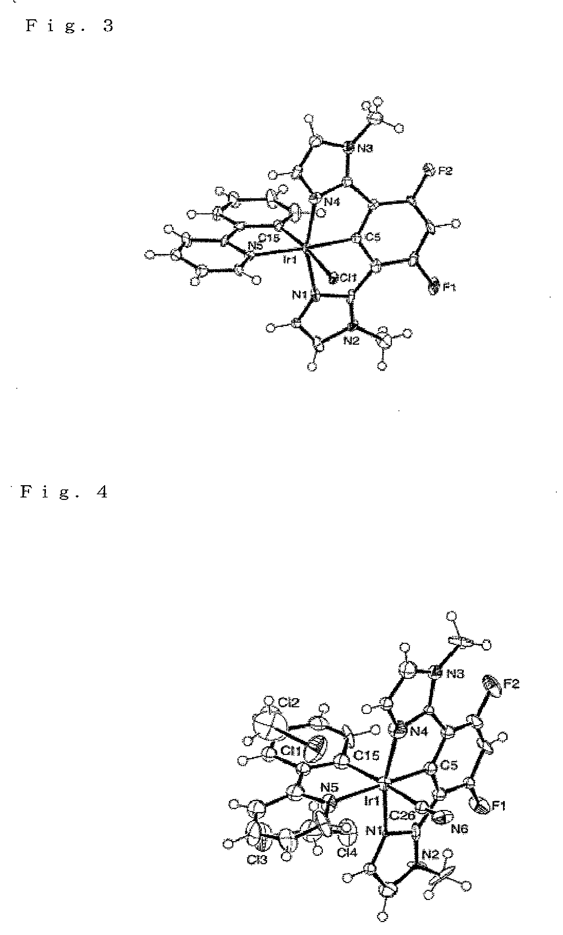

Into an eggplant flask having a capacity of 50 milliliter, **(Meifb)** IrCl₂ in an amount of 0.528 millimole (0.575 g), 2-phenylpyridine in an amount of 1.27 millimole (0.197 g) and ethylene glycol in an amount of 60 milliliter were placed, and the resultant solution was refluxed with heating being exposed with microwave irradiation for 1 minute in 3 times under an atmospheric nitrogen gas flow. The reacted solution was cooled by being left standing and then, pouring pure water, a supernatant liquid was collected by means of a centrifugal separator. Adding saturated sodium chloride solution into the supernatant liquid, a generated precipitation was separated by filtration and washed with uses of hexane and diethyl ether. Carrying out a silica column purification (dichloro-methane / methanol= 25/1), a yellow crystal of the aimed substance in an amount of 0.349 g (the yield: 50 %) was obtained.
It was confirmed in accordance with a measurement of light emission spectrum about Metal-Complex Compound 3 obtained, that λₑₘ= 480.510 nanometers (excitation wavelength: 355 nanometers).
Further, the result of conducting X-ray crystal structure analysis about Metal-Complex Compound 3 is shown in FIG. 3.

### Synthesis Example 4 (Synthesis of Metal-Complex Compound 4)

Into an eggplant flask having a capacity of 50 milliliter, Metal-Complex Compound 3 in an amount of 30.957 millimole (0.627 g), potassium cyanide in an amount of 4.78 millimole (0.311 g) and ethylene glycol in an amount of 60 milliliter were placed, and the resultant solution was refluxed with heating being exposed with microwave irradiation for 1 minute in 3 times under an atmospheric nitrogen gas flow. After cooling the reacted solution by leaving it standing, a saturated sodium chloride solution in an amount of 200 milliliter was added and the precipitate was collected by filtration. Washing the precipitate with uses of hexane and diethyl ether, a silica column purification (dichloro-methane / methanol =25/1) was carried out and a yellow crystal of the aimed substance in an amount of 0.444 g (the yield: 71 %) was obtained.
It was confirmed in accordance with a measurement of light emission spectrum about Metal-Complex Compound 4 obtained, that λₑₘ= 463.494 nanometers (excitation wavelength: 355 nanometers).
Further, the result of conducting X-ray crystal structure analysis about Metal-Complex Compound 4 is shown in FIG. 4.

### Synthesis Example 5 (Synthesis of Metal-Complex Compound 46)

The route for synthesis of the above Metal-Complex Compound 46 is illustrated as follows:

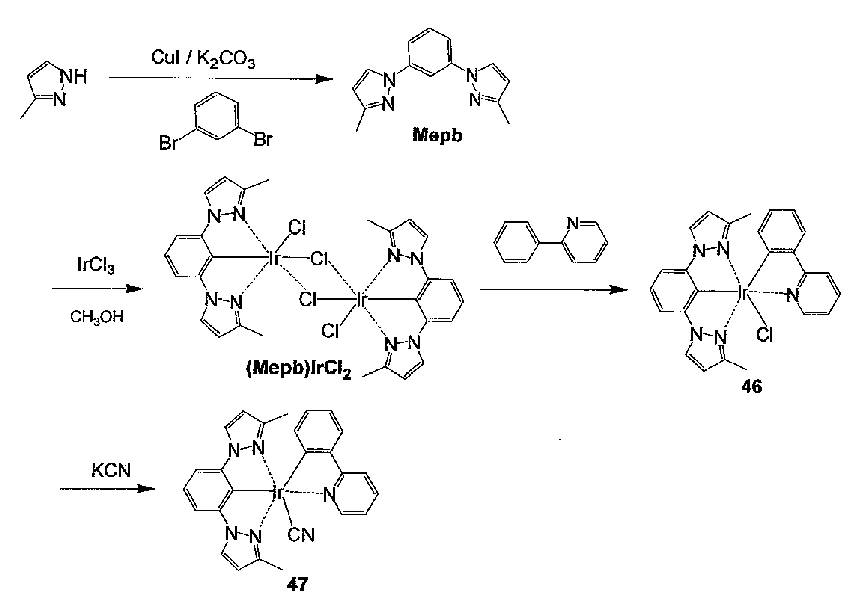

### (1) Synthesis of Mepb

Into Schlenk tube having a capacity of 50 milliliter, potassium carbonate in an amount of 43 millimole (6.0 g) heated at a temperature of 100 °C and under vacuum for 1 hour, 1,3-dibromobenzene in an amount of 10 millimole (2.56 g), 3-methylpyrazole in an amount of 32 millimole (2.6 g), copper iodide in an amount of 1.5 millimole (0.285 g) and trans-1,2-cyclohexanediamine in an amount of 6 millimole (0.85 g) were placed and a nitrogen replacement was conducted twice under a pressure reduction. The mixture was heated at a temperature of 110 °C for 24 hours. After cooling down, the mixture was diluted with a use of ethyl acetate and then, insolubles were separated by filtration. Distilling the solvent from the filtrate and carrying out a silica column purification (hexane / ethyl acetate= 5/1), a colorless oil as an aimed substance in an amount of 1.13 g (the yield: 47 %) was obtained. The colorless oil was identified as the aimed substance in accordance with a following ¹H-NMR and measurement of Electrospray Ionization Mass Spectrometry (ESI-MS).
¹H-NMR (500MHz, CDCl₃) δ 7.92(t,1H),7.82(t,2H),7.46(t,2H), 7.38(m,1H),6.18(t,2H),2.31(s,6H).
ESI-MS m/z 239.0138 (239.1297 required for [C₁₄H₁₅N₄]⁺).

### (2) Synthesis of (Mepb) IrCl₂

Placing iridium chloride trihydrate in an amount of 3.9 millimole (1.4 g), **Mepb** in an amount of 4.76 millimole (1.13 g) and methanol into an eggplant flask having a capacity of 100 milliliter, the resultant solution was refluxed overnight. The precipitate after reflux was separated by filtration, washed with uses of methanol and ether and after drying, 1.08 g (the yield: 28 %) of an aimed substance was obtained.

### (3) Synthesis of Metal-Complex Compound 46

Into an eggplant flask having a capacity of 50 milliliter, **(Mepb)** IrCl₂ in an amount of 0.25 millimole (0.255 g), 2-phenylpyridine in an amount of 0.75 millimole (0.116 g) and glycerol in an amount of 30 milliliter were placed, and the resultant solution was refluxed with heating being exposed with microwave irradiation for 4 minute and a half under an atmospheric nitrogen gas flow. After the resultant solution was cooled by leaving it standing, pure water and saturated sodium chloride solution in an amount of 50 milliliter was added, and a generated precipitation was separated by filtration and washed with a use of diethyl ether. A resultant yellow solid was re-crystallized with uses of methylene chloride and hexane, and 0.10 g (the yield: 80 %) of a yellow crystal as an aimed substance was obtained.

### Synthesis Example 6 (Synthesis of Metal-Complex Compound 47)

Into an eggplant flask having a capacity of 50 milliliter, Metal-Complex Compound 46 in an amount of 0.15 millimole (0.095 g), potassium cyanide in an amount of 6.14 millimole (0.4 g) and ethylene glycol in an amount of 30 milliliter were placed, and the resultant solution was refluxed with heating being exposed with microwave irradiation for 3 minutes under an atmospheric nitrogen gas flow. After the resultant solution was cooled by leaving it standing, pure water in an amount of 50 milliliter and saturated sodium chloride solution were added and the resultant solution was extracted with a use of methylene chloride. Distilling the solvent from the extract, a resultant crystal was washed with uses of water and ether. The crystal was identified as an aimed substance in accordance with a following ¹H-NMR measurement.
¹H-NMR(500MHz,CDCl₃), δ 10.24(d,1H),7.96(d,1H),7.88(t,1H), 7.82(m,2H),7.58(d,1H),7.34(t,1H),7.10(d,1H),6.77(t,1H),6.62(t,1H),5.95(s,1 H),5.94(d,1H),1.42(s,6H).
It was confirmed in accordance with a measurement of light emission spectrum about Metal-Complex Compound 47 obtained, that λₑₘ= 477.503 nanometers (excitation wavelength: 355 nanometers).

### Synthesis Example 7 (Metal-Complex Compound 48)

The route for synthesis of the above Metal-Complex Compound 48 is illustrated as follows:

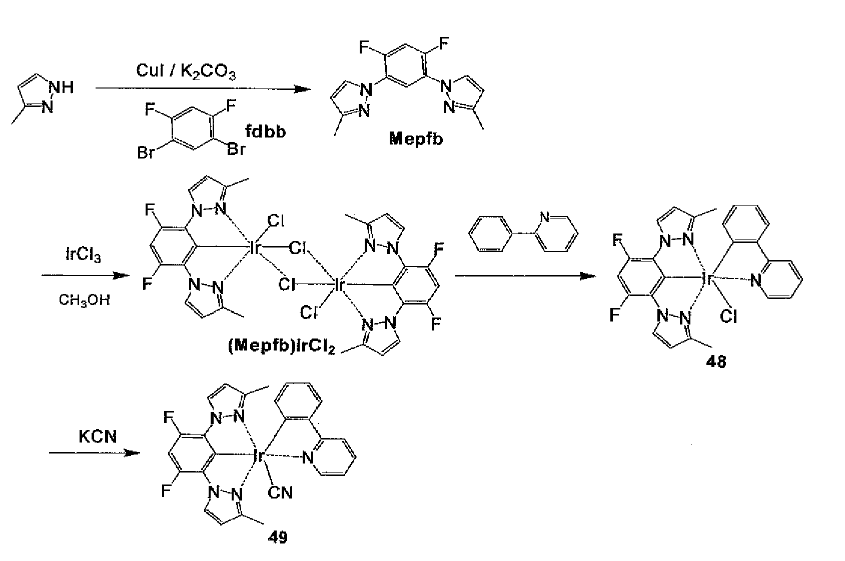

### (1) Synthesis of Mepfb

Into Schlenk tube having a capacity of 50 milliliter, potassium carbonate in an amount of 42 millimole (5.8 g) heated at a temperature of 100 °C and under vacuum for 1 hour, **Mepfb** in an amount of 9 millimole (2.65 g), 3-methylpyrazole in an amount of 40 millimole (3.3 g), copper iodide in an amount of 1.7 millimole (0.33 g) and trans-1,2-cyclohexanediamine in an amount of 6 millimole (0.85 g) were placed and a nitrogen replacement was conducted twice under a pressure reduction- The mixture was heated at a temperature of 110 °C for 24 hours. After cooling down, the mixture was diluted with a use of ethyl acetate and then, insolubles were separated by filtration. Distilling the solvent from the filtrate and carrying out a silica column purification (hexane / ethyl acetate= 10/1), a green crystal as an aimed substance in an amount of 0.23 g (the yield: 9 %) was obtained. The crystal was identified as an aimed substance in accordance with a following ¹H-NMR measurement.
¹H-NMR(500MHz,CDCl₃) δ 8.30(t,1H),7.75(t,2H),7.05(t,1H),6.18(s,2H), 2.22(s,6H)

### (2) Synthesis of (Mepfb) IrCl₂

Placing iridium chloride trihydrate in an amount of 0.625 millimole (0.22 g), **Mepfb** in an amount of 0.75 millimole (0.2 g) and methanol into an eggplant flask having a capacity of 100 milliliter, the resultant solution was refluxed overnight. The precipitate after reflux was separated by filtration, washed with uses of methanol and ether and after drying, 0.2285 g (the yield: 34 %) of an aimed substance was obtained.

### (3) Synthesis of Metal-Complex Compound 48

Into an egg plant type flask having a capacity of 50 milliliter, **(Mepfb)** IrCl₂ in an amount of 0.21 millimole (0.23 g), 2-phenylpyridine in an amount of 0.75 millimole (0.116 g) and glycerol in an amount of 30 milliliter were placed, and the resultant solution was refluxed with heating being exposed with microwave irradiation for 9 minutes under an atmospheric nitrogen gas flow. After the resultant solution was cooled by leaving it standing, pure water and saturated sodium chloride solution in an amount of 50 milliliter was added, and a generated precipitation was separated by filtration and washed with a use of hexane and ether. A resultant yellow solid was re-crystallized with uses of methylene chloride and hexane, and 0.22 g (the yield: 80 %) of a yellowish green crystal as an aimed substance was obtained. The crystal was identified as an aimed substance in accordance with a following ¹H-NMR measurement.
¹H-NMR (CDCl₃,500MHz) δ (ppm):10.26(d,1H),7.97(d,1H),7.94(d,2H), 7.87(t,1H), 7.56(d,1H),7.42(t,1H),6.81(t,1H),6.74(t,1H),6.61(t,1H), 6.02(d,1H),5.97(d,1H), 1.55(s,6H).
Further, it was confirmed in accordance with a measurement of light emission spectrum about Metal-Complex Compound 48 obtained, that λₑₘ= 475.510 nanometers (excitation wavelength: 355 nanometers).

### Synthesis Example 8 (Synthesis of Metal-Complex Compound 49)

Into an eggplant flask having a capacity of 50 milliliter, Metal-Complex Compound 48 in an amount of 0.26 millimole (0.17 g), potassium cyanide in an amount of 58 millimole (0.038 g) and ethylene glycol in an amount of 30 milliliter were placed, and the resultant solution was refluxed with heating being exposed with microwave irradiation for 3 minutes under an atmospheric nitrogen gas flow. After the resultant solution was cooled by leaving it standing, pure water in an amount of 50 milliliter and saturated sodium chloride solution were added and the resultant solution was washed with a use of hexane. A resultant solid was re-crystallized with uses of methylene chloride and hexane, and 0.043 g (the yield: 26 %) of a yellow crystal as an aimed substance was obtained. The crystal was identified as an aimed substance in accordance with following ¹H-NMR and IR measurement.
¹H-NMR (500MHz,CDCl₃) δ (ppm): 10.19(d,1H),7.97(m,3H),7.97(t,1H), 7.61(d,1H), 7.37(t,1H),6.85(m,2H),6.70(t,1H),6.02(d,1H),5.98(d,2H), 1.55(s,6H). IR (KBr pellet): 2115.41 cm⁻¹.
It was confirmed in accordance with a measurement of light emission spectrum about Metal-Complex Compound 49 obtained, that λₑₘ= 469.500 nanometers (excitation wavelength: 355 nanometers).

### Synthesis Example 9 (Metal-Complex Compound 60)

The route for synthesis of the above Metal-Complex Compound 60 and Metal-Complex Compound 61 below is illustrated as follows:

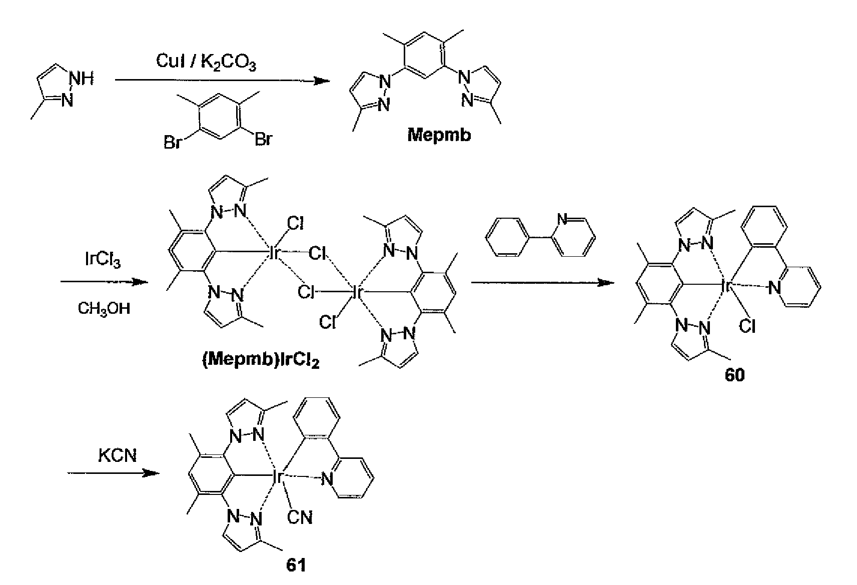

### (1) Synthesis of Mepmb

Into Schlenk tube having a capacity of 50 milliliter, potassium carbonate in an amount of 42 millimole (5.8 g) heated at a temperature of 100 °C and under vacuum for 1 hour, 1,5-dibromo-2,4-dimethylbenzene in an amount of 10.8 millimole (2.86 g), 3-methylpyrazole in an amount of 32 millimole (2.61 g), copper iodide in an amount of 1.42 millimole (0.27 g) and trans-1,2-cyclohexanediamine in an amount of 5.48 millimole (0.78 g) were placed and a nitrogen replacement was conducted twice under a pressure reduction. The mixture was heated at a temperature of 110 °C for 7 days. After cooling down, the mixture was diluted with a use of ethyl acetate and then, insolubles were separated by filtration. Distilling the solvent from the filtrate and carrying out a silica column purification (ethyl acetate), brown oil as an aimed substance in an amount of 0.18 g (the yield: 19 %) was obtained. The oil was identified as an aimed substance in accordance with following ¹H-NMR and ESI-MS measurement.
¹H-NMR (500MHz,CDCl₃) δ 7.46(d,2H),7.24(s,1H),7.13(s,1H),6.11(d,2H), 2.25(s,6H), 2.17(s,6H). ESI-MS m/z 266.9518 (267.1610 required for [C₁₆H₁₉N₄]⁺).

### (2) Synthesis of (Mepmb) IrCl₂

Placing iridium chloride trihydrate in an amount of 0.56 millimole (0.20 g), **Mepmb** in an amount of 0.68 millimole (0.18 g) and methanol into an eggplant flask having a capacity of 100 milliliter, the resultant solution was refluxed overnight. The precipitate after reflux was separated by filtration, washed with uses of methanol and ether and after drying, 0.12 g (the yield: 39 %) of an aimed substance was obtained.

### (3) Synthesis of Metal-Complex Compound 60

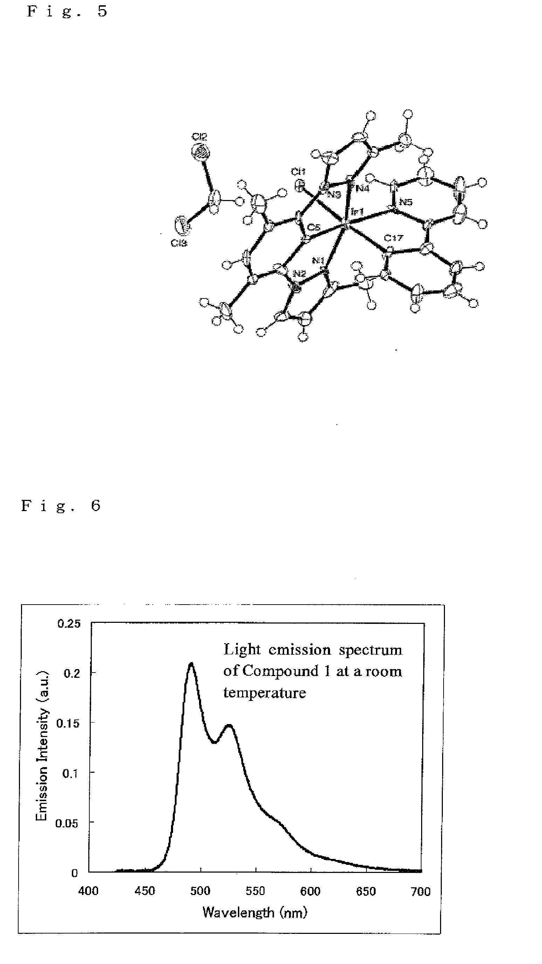

Into an eggplant flask having a capacity of 50 milliliter, **(Mepmb)**IrCl₂ in an amount of 0.11 millimole (0.12 g), 2-phenylpyridine in an amount of 0.27 millimole (0.042 g) and glycerol in an amount of 30 milliliter were placed, and the resultant solution was refluxed with heating being exposed with microwave irradiation for 9 minutes under an atmospheric nitrogen gas flow. After the resultant solution was cooled by leaving it standing, pure water and saturated sodium chloride solution in an amount of 50 milliliter was added, and a generated precipitation was separated by filtration and washed with uses of hexane and ether. A resultant yellow solid was re-crystallized twice with uses of methylene chloride and hexane, and 0.12 g (the yield: 84 %) of an yellowish green crystal as an aimed substance was obtained. The crystal was identified as an aimed substance in accordance with a following ¹H-NMR measurement.
¹H-NMR (CDCl₃,500MHz) δ (ppm):10.30(d,1H),7.95(t,3H),7.82(t,1H), 7.54(d,1H), 7.38(t,1H),6.69(m,2H),6.56(t,1H),6.02(d,1H),5.95(d,2H), 2.61(s,6H),1.55(s,6H).
Further, it was confirmed in accordance with a measurement of light emission spectrum about Metal-Complex Compound 60 obtained, that λₑₘ= 469.505 nanometers (excitation wavelength: 355 nanometers).
Further, the result of conducting X-ray crystal structure analysis about Metal-Complex Compound 60 is shown in FIG. 5.

### Synthesis Example 10 (Synthesis of Metal-Complex Compound 61)

Into an eggplant flask having a capacity of 50 milliliter, Metal-Complex Compound 60 in an amount of 0.1 millimole (0.065 g), potassium cyanide in an amount of 0.2 millimole (0.013 g) and ethylene glycol in an amount of 30 milliliter were placed, and the resultant solution was stirred while heating being exposed with microwave irradiation for 5 minutes under an atmospheric nitrogen gas flow. After the resultant solution was cooled by leaving it standing, saturated sodium chloride solution was added, and a generated precipitation was washed with uses of hexane and ether. A resultant solid was re-crystallized with uses of methylene chloride and hexane, and 0.17 g (the yield: 27 %) of a yellow crystal as an aimed substance was obtained. The crystal was identified as an aimed substance in accordance with a following ¹H-NMR measurement.
¹H-NMR (500MHz,CDCl₃) δ (ppm): 10.22(d,1H),7.98(d,2H),7.97(s,1H), 7.85(t,1H), 7.59(d,1H),7.31(t,1H),6.78(t,1H),6.75(d,1H),6.65(t,1H), 6.02(d,1H),5.96(d,2H), 2.61(s,6H), 1.54(s,6H).
It was confirmed in accordance with a measurement of light emission spectrum about Metal-Complex Compound 61 obtained, that λₑₘ= 477.503 nanometers (excitation wavelength: 355 nanometers).

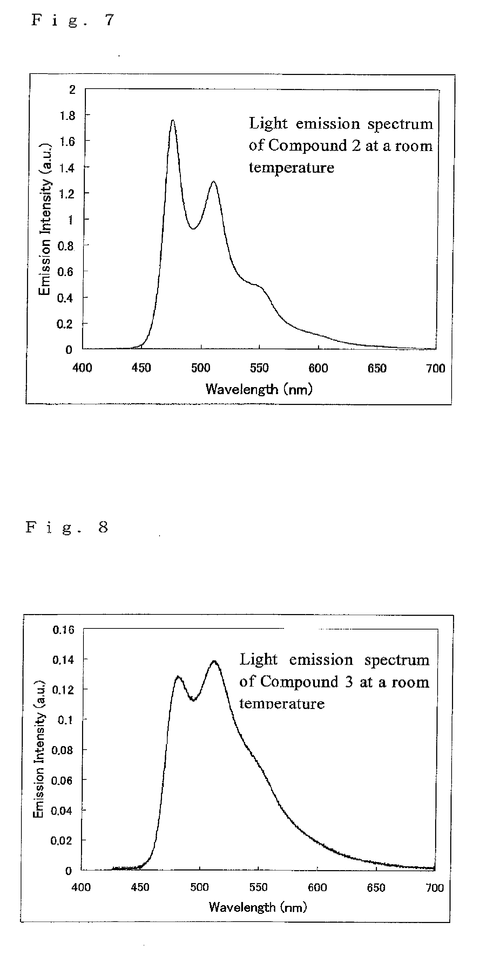

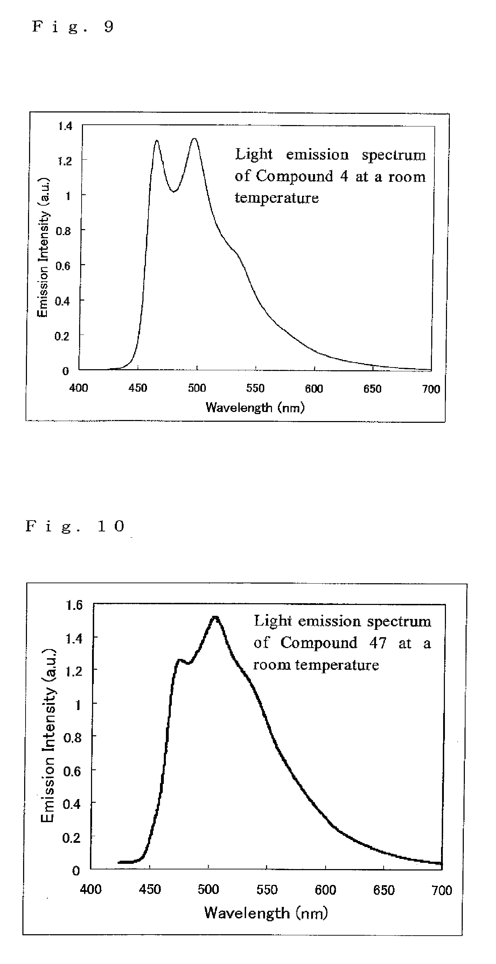

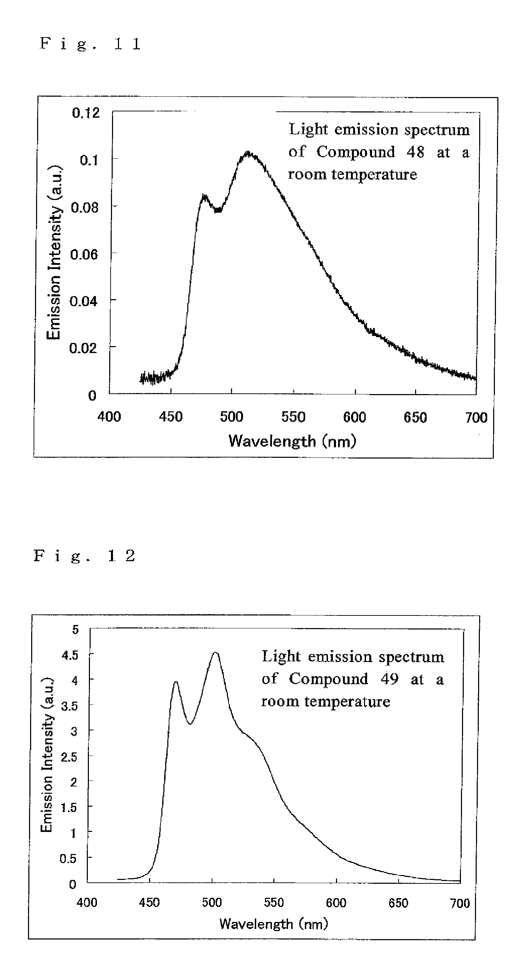

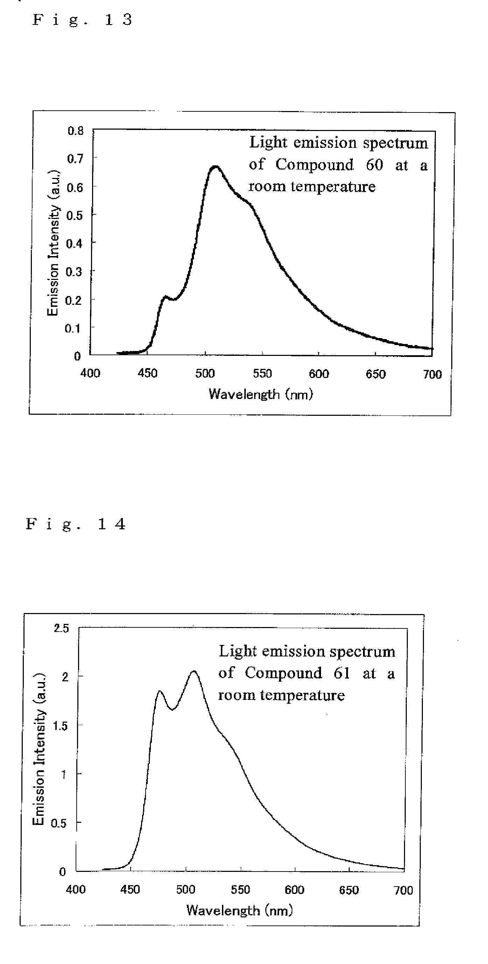

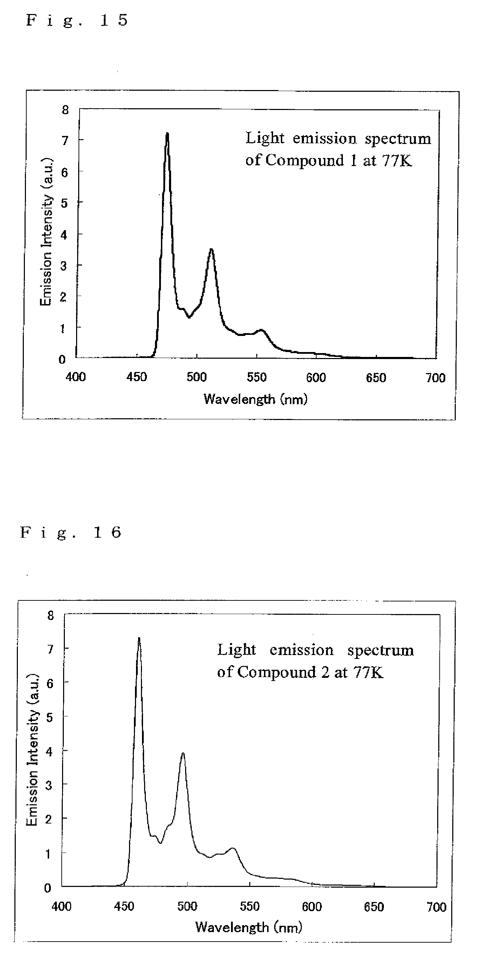

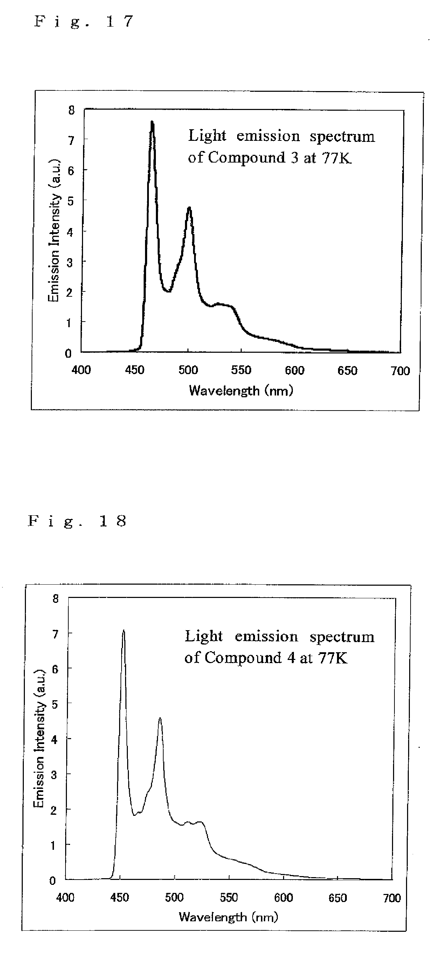

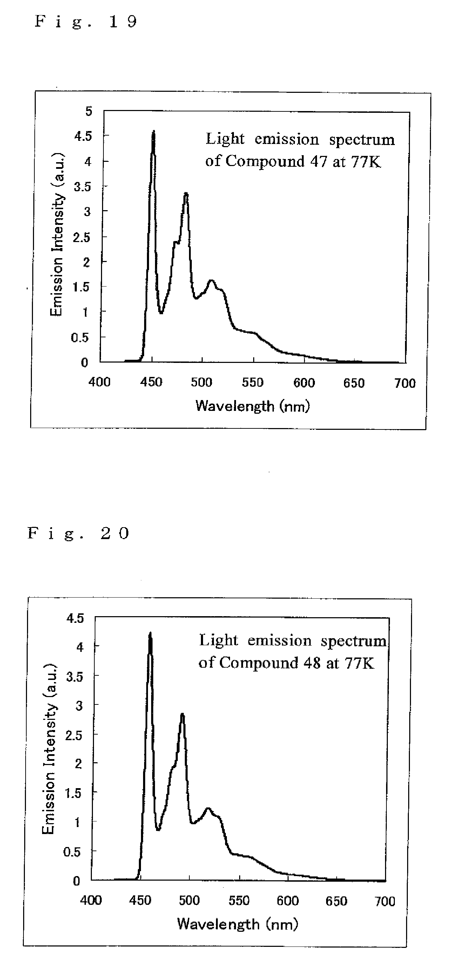

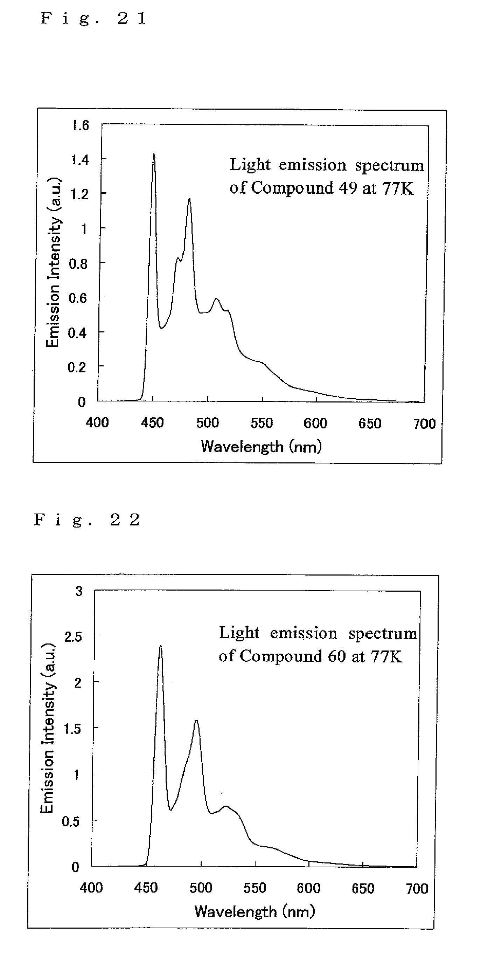

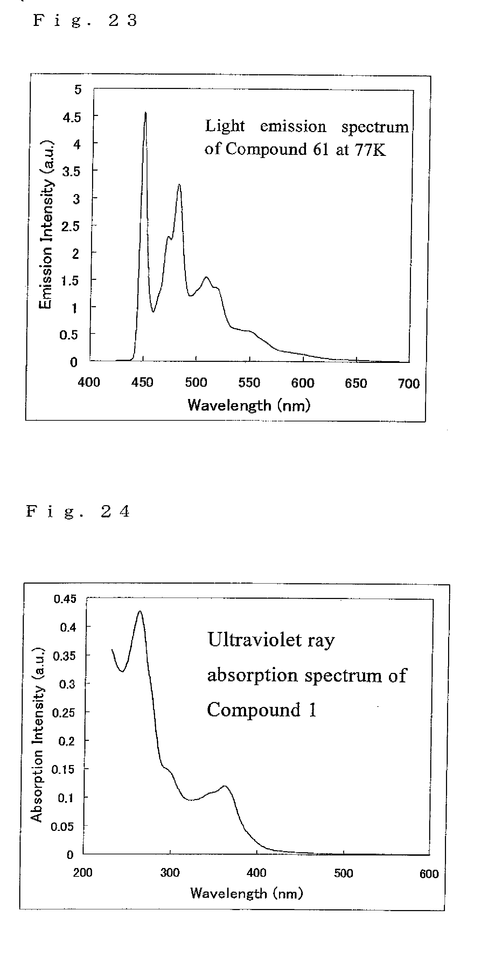

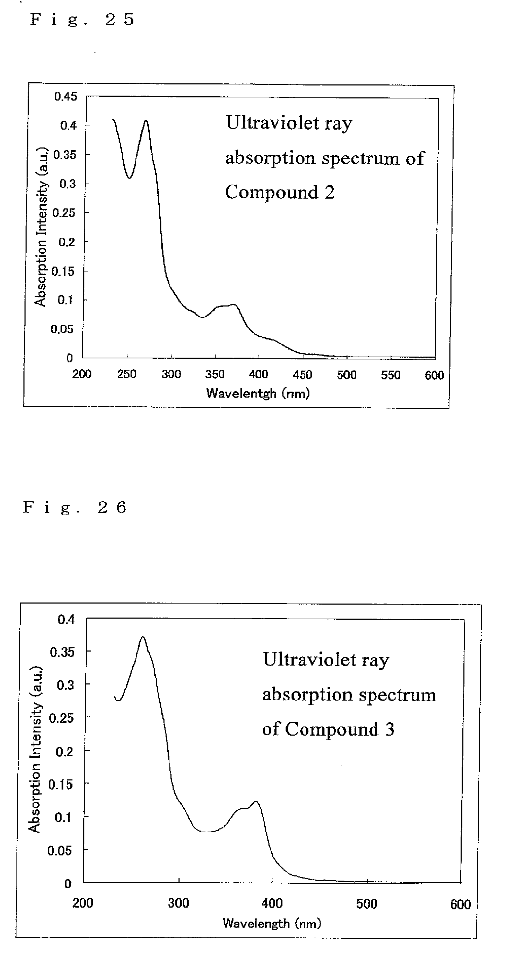

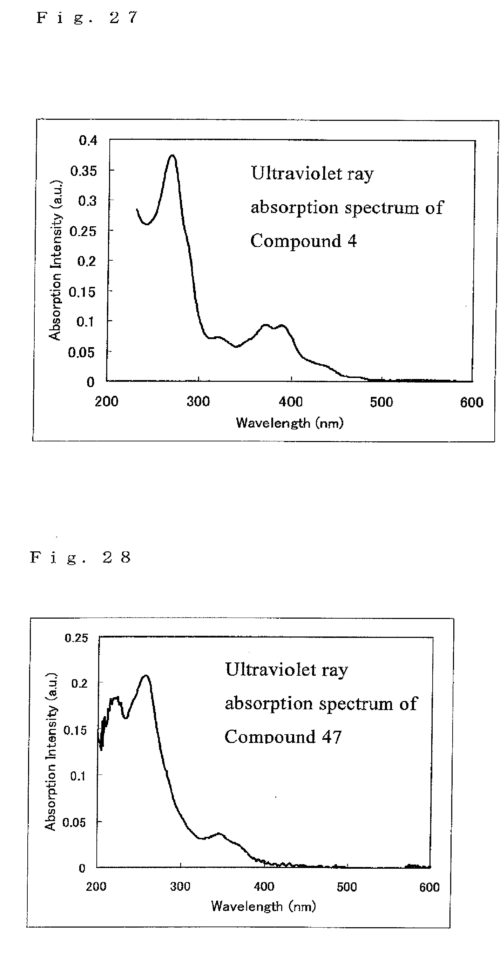

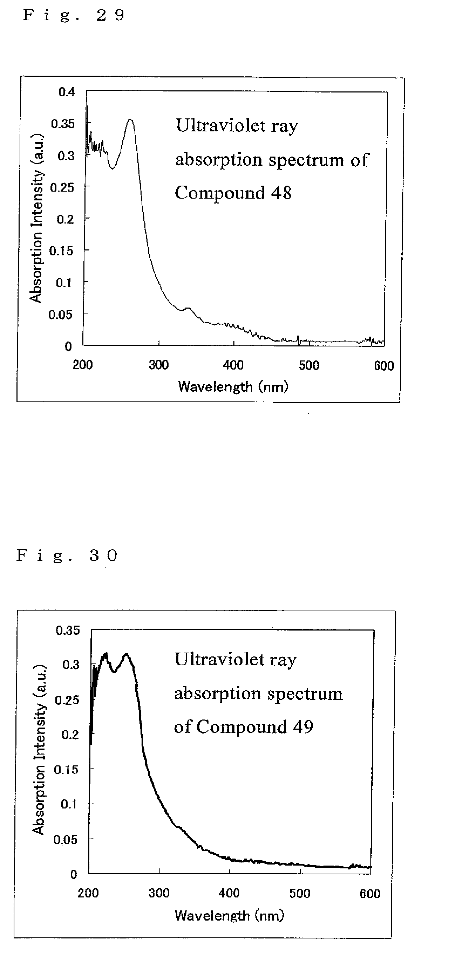

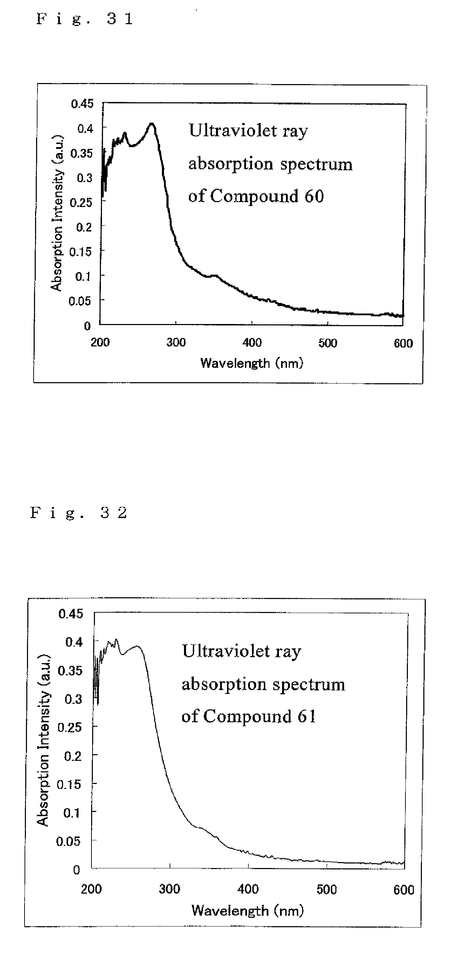

Phosphorescent Light emission spectrums of Metal-Complex Compounds 1, 2, 3, 4, 47, 48, 49, 60 and 61 at room temperatures (see FIGS. 6 to 14), at low temperatures (see FIGS. 15 to 23) and their Ultraviolet Ray absorption spectrums (see FIGS. 24 to 32) are shown in the drawings (Apparatus: F-4500 type spectrophotofluorometer, Measurement solvent: methylene chloride). Further, electrochemical values (in accordance with a cyclic voltammetry) of physical property about electrophospholescence are shown in Tables 1 and 2 below. Additionally, Kᵣ means radiation velocity and τ means lifetime of light emission respectively in Table 1. Further, ΔE1 means oxidation potential and ΔE2 means its difference with each reduction potential respectively in Table 2.

**Table 1**

| Compound | Quantum yield | ***K***ᵣ (10⁵s⁻¹) | τ (µs), Room temperature | τ (µs), 77K |
|---|---|---|---|---|
| 1 | 0.056 | 3.0 | 0.189 | 3.96 |
| 2 | 0.39 | 1.4 | 2.73 | 5.56 |
| 3 | 0.045 | 3.2 | 0.14 | 3.41 |
| 4 | 0.44 | 1.5 | 2.9 | 6.14 |
| 47 | 0.0051 | - | - | 9.64 |
| 48 | 0.00043 | - | - | 5.96 |
| 49 | 0.0044 | - | - | 12.1 |
| 60 | 0.0044 | - | - | 3.92 |
| 61 | 0.0082 | - | - | 8.05 |

**Table 2**

| Compound | Oxidation potential (V) | Reduction potential (V) | | Δ E 1 (V) | ΔE2 (V) |
|---|---|---|---|---|---|
| | E_{1/2}^{ox} | E_{1/2}^{red} | | | |
| 47 | 0.73 | -2.60 | -2.98 | 3.33 | 3.71 |
| 48 | 0.75 | -2.58 | -3.07 | 3.33 | 3.82 |
| 49 | 0.89 | -2.52 | -3.00 | 3.41 | 3.89 |
| 60 | 0.51 | -2.67 | -3.13 | 3.18 | 3.64 |
| 61 | 0.64 | -2.64 | -3.05 | 3.28 | 3.69 |

### INDUSTRIAL APPLICABILITY

As described above in detail, the present invention provides an organic EL device which emits blue light of high purity and of short wavelength with an enhanced current efficiency. Accordingly, the present invention is applicable for a field such as various display devices, display panels, backlights, illuminating light sources, beacon lights, signboards, and interior designs, particularly suitable as display device for color displays.

## Claims

1. A metal-complex compound which comprises a tridentate chelate ligand having a partial structure represented by a following general formula (I): 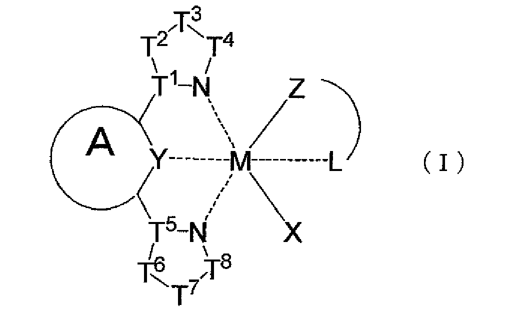 wherein **M** represents any one metal atom of Group 9 in Periodic Table;
**L** and **Z** each independently represent an organic group possessing any one atom of Groups 14 to 16 in Periodic Table respectively;
**X** represents a monovalent ligand having any one atom of Groups 14 to 17 in Periodic Table;
**Y** represents any one atom of Groups 14 to 16 in Periodic Table;
**A** represents a cycloalkane moiety having 5 to 20 carbon atoms and further may have a substituent, an aromatic hydrocarbon group having 6 to 20 carbon atoms and further may have a substituent or a heterocyclic group having 2 to 20 carbon atoms and further may have a substituent indicating that a circle enclosing the sign A shows a ring structure comprising **Y;**
**T¹** to **T⁸** each independently represents a carbon atom or a nitrogen atom on which a hydrogen atom, a cyano group, a nitro group, a halogen atom, an alkyl group having 1 to 12 carbon atoms and further may have a substituent, an alkylamino group having 1 to 12 carbon atoms and further may have a substituent, an arylamino group, having 6 to 20 carbon atoms and further may have a substituent, an alkoxy group having 1 to 12 carbon atoms and further may have a substituent, an alkoxy halide group having 1 to 12 carbon atoms and further may have a substituent, an aryloxy group having 6 to 20 carbon atoms and further may have a substituent, an aromatic hydrocarbon group having 6 to 20 carbon atoms and further may have a substituent, a heterocyclic group having 3 to 20 carbon atoms and further may have a substituent, an alkyl halide group having 1 to 12 carbon atoms and further may have a substituent, an alkenyl group having 2 to 12 carbon atoms and further may have a substituent, an alkynyl group having 2 to 12 carbon atoms and further may have a substituent or a cycloalkyl groups having 3 to 20 carbon atoms and further may have a substituent may bond.

2. The metal-complex compound according to Claim 1, wherein the tridentate chelate ligand is represented by any one of following general formulae (1) to (3): 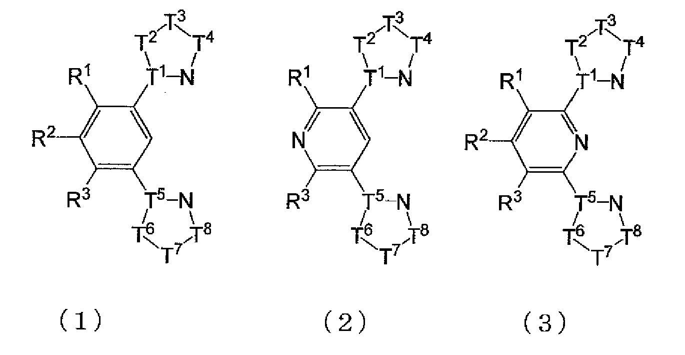 wherein T¹ to T⁸ are the same as the above description; and
R¹ to R³ each independently represents a hydrogen atom, a cyano group, a nitro group, a halogen atom, an alkyl group having 1 to 12 carbon atoms and further may have a substituent, an alkylamino group having 1 to 12 carbon atoms and further may have a substituent, an arylamino group having 6 to 20 carbon atoms and further may have a substituent, an alkoxy group having 1 to 12 carbon atoms and further may have a substituent, an alkoxy halide group having 1 to 12 carbon atoms and further may have a substituent, an aryloxy group having 6 to 20 carbon atoms and further may have a substituent, an aromatic hydrocarbon group having 6 to 20 carbon atoms and further may have a substituent, a heterocyclic group having 3 to 20 carbon atoms and further may have a substituent, an alkyl halide group having 1 to 12 carbon atoms and further may have a substituent, an alkenyl group having 2 to 12 carbon atoms and further may have a substituent, an alkynyl group having 2 to 12 carbon atoms and further may have a substituent or a cycloalkyl groups having 3 to 20 carbon atoms and further may have a substituent.

3. The metal-complex compound according to Claim 1 or 2, wherein the tridentate chelate ligand is represented by any one of following general formulae (4) to (15): 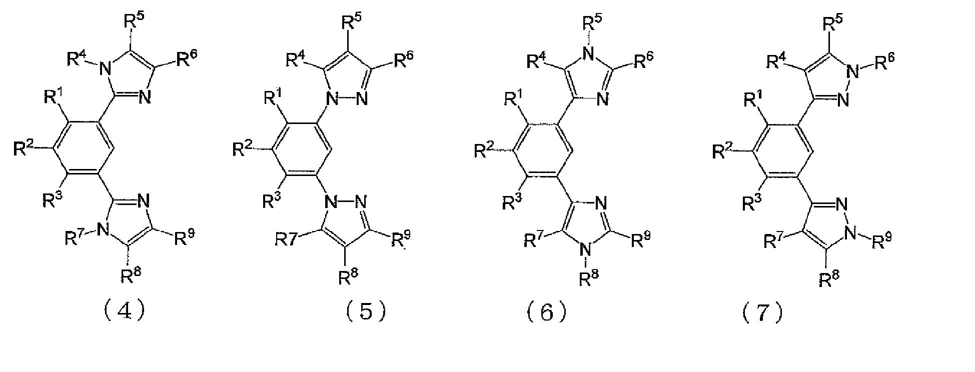 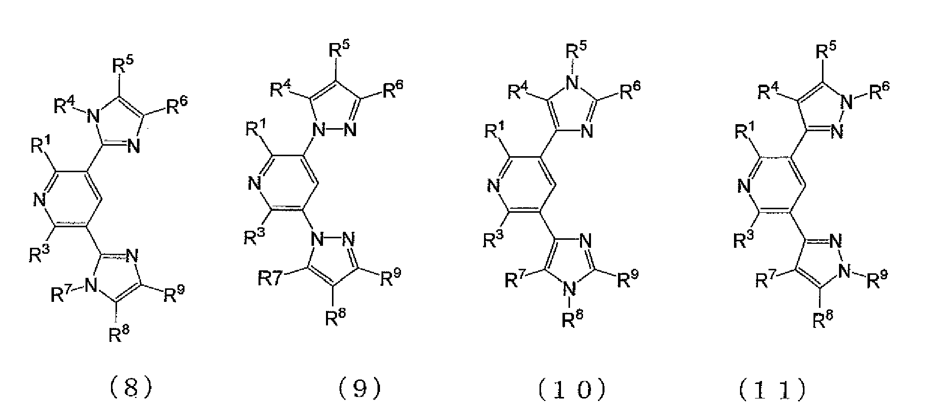 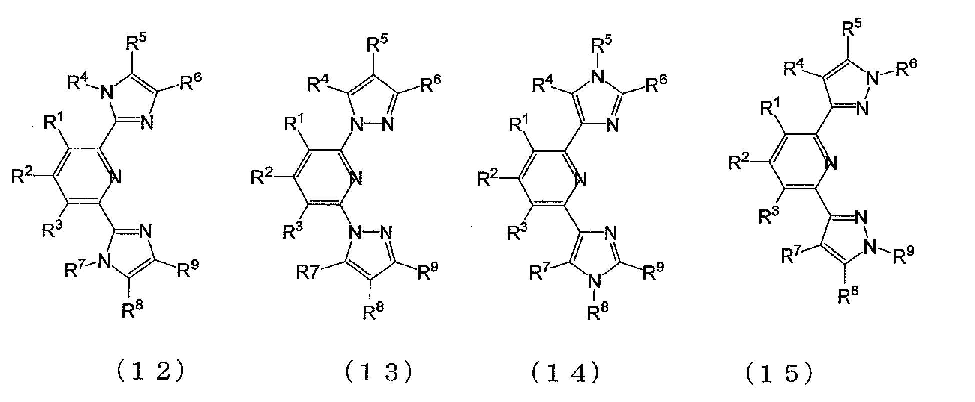 wherein R¹ to R⁹ each independently represents the same as described about the above R¹ to R³;
while R¹ to R³ never bond with each other.

4. The metal-complex compound according to Claim 1 or 2, wherein the ligand formed by L-Z is any one compound expressed by following general formulae (16) to (24): 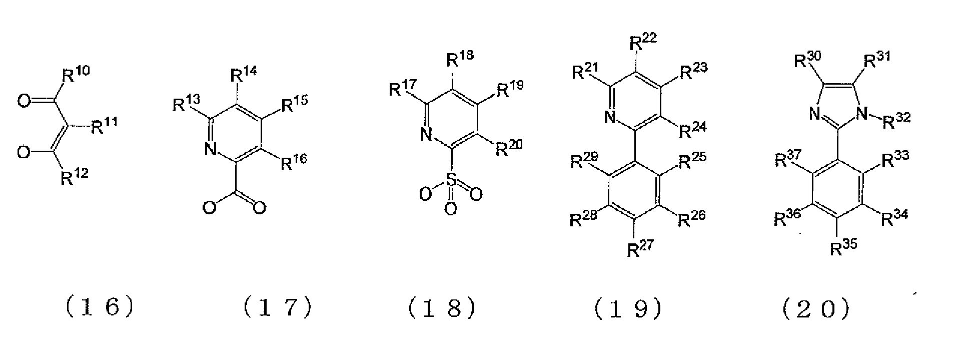 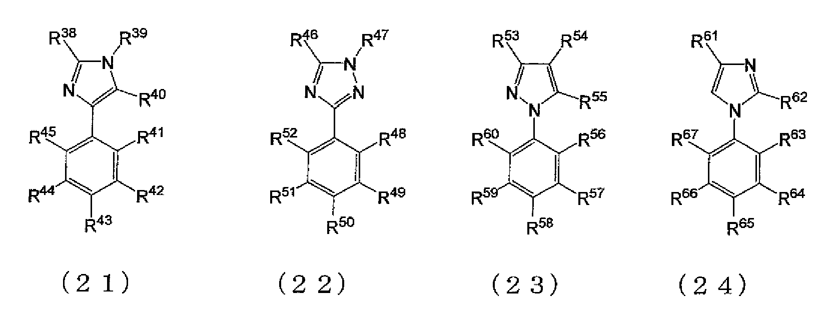 wherein R¹⁰ to R⁶⁷ each independently represents the same as described about the above R¹ to R³.

5. The metal-complex compound according to Claim 1 or 2, which is expressed by any one of following general formulae (I-1) to (I-8): 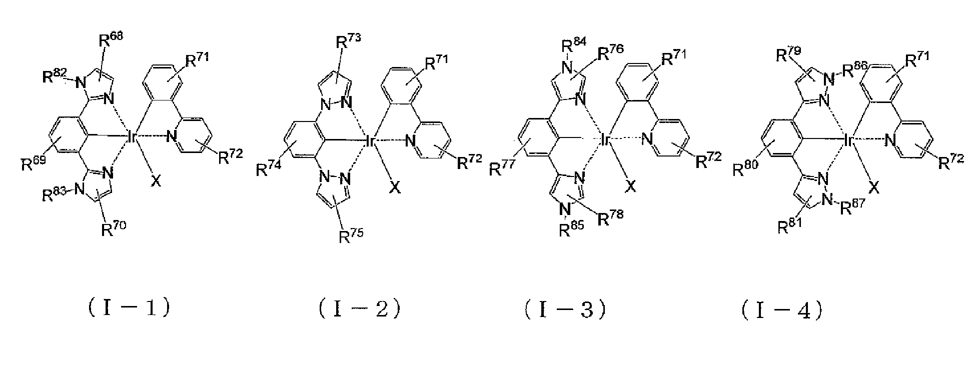 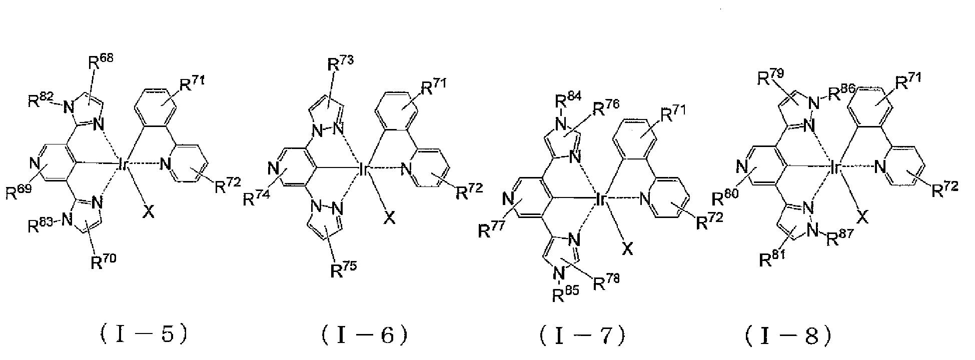 wherein R⁶⁸ to R⁸⁷ each independently represents the same as described about the above R¹ to R³; and
the number of R⁶⁸ to R⁸⁷ may be plural and when any of R⁶⁸ to R⁸⁷ exists plurally, they may be the same with or different from each other.

6. The metal-complex compound according to Claim 1 or 2, which is expressed by any one of following general formulae (I-9) to (I-12): 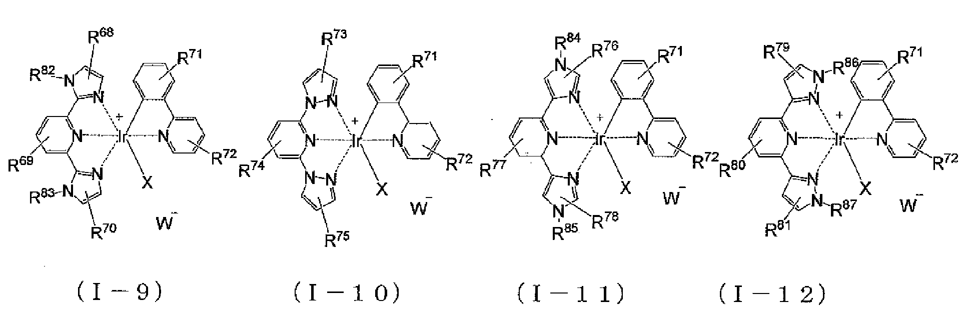 wherein W is an anion having minus one valence comprising at least one kind of metal atom among Groups 13 to 16 in Periodic Table;
R⁶⁸ to R⁸⁷ each independently represents the same as described about the above R¹ to R³; and
the number of R⁶⁸ to R⁸⁷ may be plural and when any of R⁶⁸ to R⁸⁷ exists plurally, they may be the same with or different from each other.

7. An organic electroluminescence device which comprises one or more organic thin film layers having at least one light emitting layer sandwiched between a pair of electrodes, wherein the organic thin film layer comprises the metal-complex compound according to Claim 1, which emits light by applying an electric voltage between the pair of electrodes.

8. The organic electroluminescence device according to Claim 7, wherein said metal-complex compound is contained in the light emitting layer.

9. The organic electroluminescence device according to Claim 7, which emits bluish light.

10. The organic electroluminescence device according to Claim 7, wherein said organic thin film layer comprising the metal-complex compound is formed by coating process.
